(19) **Europäisches Patentamt**
**European Patent Office**
**Office européen des brevets**

(11) **EP 1 487 577 B1**

(12) **EUROPÄISCHE PATENTSCHRIFT**

(45) Veröffentlichungstag und Bekanntmachung des Hinweises auf die Patenterteilung:
**01.07.2009 Patentblatt 2009/27**

(21) Anmeldenummer: **03709774.8**

(22) Anmeldetag: **12.03.2003**

(51) Int Cl.:
***B01J 27/198*** *(2006.01)*     ***C07C 51/215*** *(2006.01)*
***C07C 51/25*** *(2006.01)*

(86) Internationale Anmeldenummer:
**PCT/EP2003/002506**

(87) Internationale Veröffentlichungsnummer:
**WO 2003/078058 (25.09.2003 Gazette 2003/39)**

(54) **KATALYSATOR-PRECURSOR FÜR DIE HERSTELLUNG VON MALEINSÄUREANHYDRID UND VERFAHREN ZU DESSEN HERSTELLUNG**

CATALYST-PRECURSOR FOR THE PRODUCTION OF MALEIC ACID ANHYDRIDE AND METHOD FOR THE PRODUCTION THEREOF

PRECURSEUR DE CATALYSEUR PERMETTANT LA PREPARATION D'ANHYDRIDE D'ACIDE MALEIQUE ET PROCEDE POUR LE PREPARER

(84) Benannte Vertragsstaaten:
**AT BE BG CH CY CZ DE DK EE ES FI FR GB GR HU IE IT LI LU MC NL PT RO SE SI SK TR**

(30) Priorität: **15.03.2002 DE 10211445**

(43) Veröffentlichungstag der Anmeldung:
**22.12.2004 Patentblatt 2004/52**

(73) Patentinhaber: **BASF SE**
**67056 Ludwigshafen (DE)**

(72) Erfinder:
• **WEIGUNY, Jens**
**Shanghai 200336 (CN)**
• **STORCK, Sebastian**
**68167 Mannheim (DE)**
• **DUDA, Mark**
**67071 Ludwigshafen (DE)**
• **DOBNER, Cornelia**
**67227 Frankenthal (DE)**

(56) Entgegenhaltungen:
WO-A-00/72963     US-A- 4 670 415
US-A- 5 364 824     US-A- 5 922 637

Bemerkungen:
Die Akte enthält technische Angaben, die nach dem Eingang der Anmeldung eingereicht wurden und die nicht in dieser Patentschrift enthalten sind.

**EP 1 487 577 B1**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft einen Vanadium, Phosphor und Sauerstoff enthaltenden Katalysator-Precursor sowie ein Verfahren zu dessen Herstellung für die Herstellung von Maleinsäureanhydrid durch heterogenkatalytische Gasphasenoxidation eines Kohlenwasserstoffs mit mindestens vier Kohlenstoffatomen.

Des weiteren betrifft die vorliegende Erfindung einen Vanadium, Phosphor und Sauerstoff enthaltenden Katalysator sowie ein Verfahren zu dessen Herstellung unter Einsatz des erfindungsgemäßen Katalysator-Precursors.

Ferner betrifft die vorliegende Erfindung ein Verfahren zur Herstellung von Maleinsäureanhydrid durch heterogenkatalytische Gasphasenoxidation eines Kohlenwasserstoffs mit mindestens vier Kohlenstoffatomen unter Einsatz des erfindungsgemäßen Katalysators.

Maleinsäureanhydrid ist ein wichtiges Zwischenprodukt bei der Synthese von γ-Butyrolacton, Tetrahydrofuran und 1,4-Butandiol, welche ihrerseits als Lösungsmittel eingesetzt werden oder beispielsweise zu Polymeren, wie Polytetrahydrofuran oder Polyvinylpyrrolidon weiterverarbeitet werden.

Die Herstellung von Maleinsäureanhydrid durch Oxidation von Kohlenwasserstoffen wie n-Butan, n-Butenen oder Benzol an geeigneten Katalysatoren ist schon seit langem bekannt. Im Allgemeinen werden hierzu Vanadium-, Phosphor- und Sauerstoff enthaltende Katalysatoren (sogenannte VPO-Katalysatoren) eingesetzt (siehe Ullmann's Encyclopedia of Industrial Chemistry, 6th edition, 2000 electronic release, Chapter "MALEIC AND FUMARIC ACIDS, Maleic Anhydride - Production").

Die im Allgemeinen eingesetzten Vanadium-, Phosphor- und Sauerstoff enthaltenden Katalysatoren werden in der Regel wie folgt hergestellt:

(1) Synthese eines Vanadylphosphat-Hemihydrat-Precursors ($VOHPO_4 \cdot \frac{1}{2}H_2O$) aus einer fünfwertigen Vanadium-Verbindung (z.B. $V_2O_5$), einer fünf- oder dreiwertigen Phosphor-Verbindung (z.B. Ortho- und/oder Pyrophosphorsäure, Phosphorsäureester oder phosphorige Säure) und einem reduzierend wirkenden Alkohol (z.B. Isobutanol), Isolierung des Niederschlags und Trocknung, gegebenenfalls Formgebung (z.B. Tablettierung); und

(2) Präformierung zum Vanadylpyrophosphat ($(VO)_2P_2O_7$) durch Kalzinierung.

Durch den Einsatz eines reduzierend wirkenden Alkohols als Reduktionsmittel verbleiben im gebildeten Precusor-Niederschlag im Allgemeinen mehrere Gew.-% an organischen Verbindungen eingeschlossen, welche sich auch durch sorgsames Waschen nicht entfernen lassen. Diese üben bei der weiteren Katalysatorherstellung, insbesondere bei der Kalzinierung, einen negativen Effekt auf die katalytischen Eigenschaften des Katalysators aus. So besteht bei der späteren Kalzinierung die Gefahr der Verdampfung beziehungsweise der thermischen Zersetzung dieser eingeschlossenen organischen Verbindung unter Bildung gasförmiger Komponenten, welche zu einem Druckanstieg im Inneren und somit zu einer Zerstörung der Katalysatorstruktur führen können. Dieser nachteilige Effekt ist besonders stark ausgeprägt bei der Kalzinierung unter oxidativen Bedingungen, da durch die Bildung der oxidierten Abbauprodukte, wie beispielsweise Kohlenmonoxid oder Kohlendioxid, eine wesentlich größere Gasmenge gebildet wird. Des Weiteren entstehen bei der Oxidation dieser organischen Verbindungen lokal sehr große Wärmemengen, welche zu einer thermischen Schädigung des Katalysators führen können.

Ferner besitzen die eingeschlossenen organischen Verbindungen auch einen signifikanten Einfluss auf die Einstellung der lokalen Oxidationsstufe des Vanadiums. So belegen B. Kubias et al. in Chemie Ingenieur Technik 72 (3), 2000, Seiten 249 bis 251 den reduzierenden Effekt organischen Kohlenstoffs bei der anaeroben Kalzinierung (unter nicht-oxidativen Bedingungen) eines aus isobutanolischer Lösung erhaltenen Vanadylhydrogenphosphat-Hemihydrat-Precursors. Durch anaerobe Kalzinierung wurde in dem genannten Beispiel eine mittlere Oxidationsstufe des Vanadiums von 3,1 erhalten, wohingegen durch aerobe Kalzinierung (unter oxidativen Bedingungen) eine mittlere Oxidationsstufe des Vanadiums von etwa 4 erhalten wird.

EP-A 0 520 972 beschreibt die Herstellung eines Vanadylhydrogenphosphat-Hemihydrat-Precursors durch Umsetzung von Vanadiumpentoxid mit Phosphorsäure in einem organischen Medium, wie beispielsweise einem primären oder sekundären Alkohol. Der Gehalt an anhaftenden und/oder eingelagerten organischen Verbindungen wird mit bis zu 40 Gew.-% angegeben, wobei nach der Lehre der zitierten Schrift eine 8-stündige Trocknung bei 150°C zu einem Gehalt von etwa 25 Gew.-% und eine 4-stündige Trocknung (Temperung) bei 250°C zu einem Gehalt von etwa 2 Gew.-% führt.

Beispiel 1 der EP-A 0 520 972 beschreibt die Herstellung des Precursors durch Umsetzung von Vanadiumpentoxid mit 105,7 %iger Phosphorsäure in Gegenwart von Oxalsäure in Isobutanol als reduzierend wirkendes organisches Medium unter Erhitzen unter Rückfluß, anschließendem Abdekantieren der überstehenden Lösung, Trocknung des verbleibenden Breis bei 100 bis 150°C und nachfolgende fünfstündige Temperung bei 250 bis 260°C. Der erhaltene Katalysatorprecursor wurde anschließend mit Graphit vermischt, tablettiert und unter verschiedenen Kalzinationsbedingungen zum Vanadylpyrophosphat enthaltenden, fertigen Katalysator kalziniert. Nachteilig an der beschriebenen Herstellvorschrift ist insbesondere der hohe Gehalt anhaftender und/oder eingelagerter organischer Verbindungen im Precursor, welcher (i) durch den Einsatz von Oxalsäure, (ii) durch das Abdekantieren der überstehenden Lösung und Eindampfen des verbleibenden Breis und (iii) durch die gewählten Temperbedingungen zurückzuführen ist.

Beispiel 2 der EP-A 0 520 972 beschreibt die Herstellung des Precursors durch Umsetzung von Vanadiumpentoxid mit 100 %iger Phosphorsäure in Gegenwart von Isobutanol als reduzierend wirkendes organisches Medium unter Erhitzen unter Rückfluß, Filtration, Waschen und Trocknung bei 145°C. Der erhaltene Katalystorprecursor wurde anschließend eine Stunde bei 400°C unter Luft kalziniert, mit Graphit vermischt und zum fertigen Katalysator tablettiert. Durch die bei der Kalzinierung gewählte Temperatur von 400°C erfolgt die Phasenumwandlung zum Vanadylpyrophosphat bereits im pulverförmigen Zustand vor der Tablettierung.

WO 00/72963 lehrt die Herstellung eines Vanadylhydrogenphosphat-Hemihydrat-Precursors durch Umsetzung von Vanadiumpentoxid mit Orthophosphorsäure in Gegenwart von Isobutanol und einem Polyol (zum Beispiel einem Diol), wobei der erhaltene Niederschlag anschließend filtriert, gewaschen, bei 120 bis 200°C getrocknet, 3 Stunden bei 300°C unter Luft getempert, tablettiert sowie zur Umwandlung in die katalytisch aktive Form bei einer Temperatur von bis zu 600°C kalziniert wird. Der durch diese Maßnahmen hergestellte Precursor weist nach der dreistündigen Temperung bei 300°C einen Kohlenstoff-Gehalt von 0,7 bis 3 Gew.-% auf. Die besten katalytischen Eigenschaften werden mit Katalysatoren erreicht, welche nach der Lehre der zitierten Schrift unter Einsatz von Orthophosphorsäure in Gegenwart von Isobutanol und einem Polyol hergestellt wurden und deren Kohlenstoff-Gehalt im Precusor nach der genannten dreistündigen Temperung bei 300°C 0,8 bis 1,5 Gew.-% beträgt. Die bei Einsatz eines derartigen Katalysators erreichte Ausbeute an Maleinsäureanhydrid bei 400°C betrug etwa 30 bis 45 %. Im Gegensatz dazu zeigt ein Katalysator, welcher durch Umsetzung von Vanadiumpentoxid mit Orthophosphorsäure in Gegenwart von Isobutanol gefällt wurde und dessen Precursor nach einer fünfstündigen Trocknung bei 125°C bereits einen relativ niedrigen Kohlenstoff-Gehalt von 0,6 Gew.-% aufweist, nur eine weitaus niedrigere Ausbeute von Maleinsäureanhydrid von etwa 17 % bei 400°C.

Die Ausführungen in WO 00/72963 zeigen, dass ein niedriger Kohlenstoff-Gehalt im Precursor kein hinreichendes Kriterium für den Erhalt eines aktiven und selektiven Katalysators darstellt. Das vorgeschlagene Herstellverfahren hat den Nachteil des Einsatzes einer weiteren organischen Komponente sowie einer relativ schlechten Performance des erhältlichen Katalysators, wonach selbst bei einer Reaktionstemperatur von 400°C nur eine Maleinsäureanhydrid-Ausbeute von 30 bis 45 % erreicht wird.

EP-A 0 056 183 lehrt die Herstellung eines Vanadylhydrogenphosphat-Hemihydrat-Precursors durch Reduktion von Vanadiumpentoxid in einem reduzierend wirkenden, flüssigen Medium, Umsetzung der erhaltenen Zwischenstufe mit einer Mischung aus 45 bis 90 % Ortho-, 10 bis 50 % Pyro- und 0 bis 10 % Tri- und Polyphosphorsäure, Abtrennung des Niederschlags, Trocknung und Kalzinierung. Die Beispiele 1 bis 7 beschreiben die Herstellung des Precursors durch Umsetzung von Vanadiumpentoxid mit einer Mischung aus 49 % Ortho-, 42 % Pyro-, 8 % Tri- und 1 % Polyphosphorsäure (entspricht etwa 105 %iger Phosphorsäure) in Gegenwart von Isobutanol unter Erhitzen unter Rückfluß, Filtration und Trocknung bei 150°C. Der erhaltene Katalystorprecursor wurde anschließend eine Stunde bei 400°C unter Luft kalziniert, mit Graphit vermischt und zum fertigen Katalysator tablettiert. Durch die bei der Kalzinierung gewählte Temperatur von 400°C erfolgt die Phasenumwandlung zum Vanadylpyrophosphat bereits im pulverförmigen Zustand vor der Tablettierung.

Aufgabe der vorliegenden Erfindung war es, ein Verfahren zur Herstellung eines Vanadium, Phosphor und Sauerstoff enthaltenden Katalysator-Precursors für die Herstellung von Maleinsäureanhydrid durch heterogenkatalytische Gasphasenoxidation eines Kohlenwasserstoffs mit mindestens vier Kohlenstoffatomen zu finden, welches die oben genannten Nachteile nicht mehr besitzt, technisch einfach durchzuführen ist und nach einer technisch ebenfalls einfach durchzuführenden Präformierung zu einem partikulären Katalysator mit weitgehend homogener Oxidationsstufe des Vanadiums sowohl innerhalb der einzelnen Katalysator-Partikel als auch zwischen den verschiedenen Katalysator-Partikeln untereinander führt und welcher eine hohe Aktivität und hohe Selektivität besitzt.

Demgemäß wurde ein Verfahren zur Herstellung eines Vanadium, Phosphor und Sauerstoff enthaltenden Katalysator-Precursors für die Herstellung von Maleinsäureanhydrid durch heterogenkatalytische Gasphasenoxidation eines Kohlenwasserstoffs mit mindestens vier Kohlenstoffatomen gefunden, das dadurch gekennzeichnet ist, dass man

(a) Vanadiumpentoxid in Gegenwart eines primären oder sekundären, nichtcyclischen oder cyclischen, unverzweigten oder verzweigten, gesättigten Alkohols mit 3 bis 6 Kohlenstoffatomen mit 102 bis 110 %iger Phosphorsäure in einem Temperaturbereich von 80 bis 160°C umsetzt;

(b) den gebildeten Niederschlag isoliert;

(c) im isolierten Niederschlag durch Temperung in einem Temperaturbereich von 250 bis 350°C einen Gehalt an organischem Kohlenstoff von ≤1,1 Gew.-% einstellt, wobei das getemperte Produkt nach Zusatz von 3,0 Gew.-% Graphit als inneren Standard unter Anwendung von CuKα-Strahlung ($\lambda$ = 1,54 · $10^{-10}$ m) ein Pulver-Röntgenbeugungsdiagramm ergibt, das im $2\theta$-Bereich ein Peakhöhenverhältnis des Peaks einer eventuell vorhandenen Pyrophosphatphase bei 28,5° zu dem vom Graphit stammenden Peak bei 26,6° von ≤0,1 aufweist; und

(d) das aus Schritt (c) erhaltene, getemperte Produkt zu Partikeln mit einem gemittelten Durchmesser von mindestens 2 mm formt.

Wesentlich beim erfindungsgemäßen Verfahren sind:

- der Einsatz von 102 bis 110 %iger Phosphorsäure,

- der Einsatz eines primären oder sekundären, nichtcyclischen oder cyclischen, unverzweigten oder verzweigten, gesättigten Alkohols mit 3 bis 6 Kohlenstoffatomen,

- die Einstellung eines Gehalts an organischem Kohlenstoff von ≤1,1 Gew.-% durch Temperung unter geeigneten Bedingungen unter weitgehender Vermeidung der Ausbildung einer Pyrophosphatphase, und

- die Formgebung des getemperten und von der Pyrophosphatphase weitgehend freien Produkts.

Die obengenannten Schritte (a) bis (d) sind Im Folgenden näher erläutert:

Schritt (a)

Die beim erfindungsgemäßen Verfahren einzusetzende Phosphorsäure besitzt einen rechnerischen Gehalt an $H_3PO_4$ von 102 bis 110 Gew.-%. Sie wird vereinfacht als 102 bis 110 %ige Phosphorsäure bezeichnet. Bei der 102 bis 110 %igen Phosphorsäure handelt es sich um ein Gemisch enthaltend Orthophosphorsäure ($H_3PO_4$), Pyrophosphorsäure ($H_4P_2O_7$) und Polyphosphorsäuren der allgemeinen Formel $H_{n+2}P_nO_{3n+1}$ mit n ≥ 3. Bevorzugt wird beim erfindungsgemäßen Verfahren 102 bis 108 %ige, besonders bevorzugt 102 bis 106 %ige und ganz besonders bevorzugt 104 bis 106 %ige Phosphorsäure eingesetzt. Die einzusetzende Phosphorsäure wird im Allgemeinen durch Einbringung von Phosphorpentoxid in Wasser oder wässriger, beispielsweise 85 bis 100 %iger, Phosphorsäure, dargestellt.

Die erfindungsgemäße Maßnahme des Einsatzes von 102 bis 110 %iger Phosphorsäure führt in Kombination zu den weiteren erfindungsgemäßen Maßnahmen gegenüber dem Einsatz einer Phosphorsäure niedrigerer Konzentration, insbesondere gegenüber 85 bis 100 %iger Phosphorsäure sowie fester ortho-Phosphorsäure, überraschenderweise zur Bildung eines Katalysator-Precursors, welcher unter vergleichbaren Bedingungen zu einem Katalysator führt, welcher eine höhere Aktivität, eine höhere Selektivität zu Maleinsäureanhydrid und eine höhere Ausbeute an Maleinsäureanhydrid besitzt. Durch die höhere Aktivität ist beim Einsatz des Katalysators beispielsweise die Einstellung einer niedrigeren Reaktionstemperatur möglich.

Als reduzierend wirkende Komponente wird beim erfindungsgemäßen Verfahren ein primärer oder sekundärer, nichtcyclischer oder cyclischer, unverzweigter oder verzweigter, gesättigter Alkohol mit 3 bis 6 Kohlenstoffatomen sowie deren Gemische eingesetzt. Bevorzugt ist beim erfindungsgemäßen Verfahren der Einsatz eines primären oder sekundären, unverzweigten oder verzweigten $C_3$- bis $C_6$-Alkanols oder der Einsatz von Cyclopentanol oder Cyclohexanol. Als geeignete Alkohole seien n-Propanol (1-Propanol), Isopropanol (2-Propanol), n-Butanol (1-Butanol), sek.-Butanol (2-Butanol), Isobutanol (2-Methyl-1-propanol), 1-Pentanol, 2-Pentanol, 3-Pentanol, 2-Methyl-1-butanol, 3-Methyl-l-butanol, 3-Methyl-2-butanol, 2,2-Dimethyl-1-propanol, 1-Hexanol, 2-Hexanol, 3-Hexanol, 2-Methyl-1-hexanol, 3-Methyl-1-pentanol, 4-Methyl-1-pentanol, 3-Methyl-2-pentanol, 4-Methyl-2-pentanol, 2,2-Dimethyl-1-butanol, 2,3-Dimethyl-1-butanol, 3,3-Dimethyl-1-butanol, 3,3-Dimethyl-2-butanol, Cyclopentanol, Cyclohexanol und deren Gemische genannt. Besonders bevorzugt eingesetzt werden primäre, unverzweigte oder verzweigte $C_3$- bis $C_5$-Alkanole sowie Cyclohexanol. Ganz besonders bevorzugt sind n-Propanol (1-Propanol), n-Butanol (1-Butanol), Isobutanol (2-Methyl-1-propanol), 1-Pentanol, 2-Methyl-1-butanol, 3-Methyl-1-butanol und Cyclohexanol, insbesondere von Isobutanol.

Die erfindungsgemäße Maßnahme des Einsatzes eines primären oder sekundären, nichtcyclischen oder cyclischen, unverzweigten oder verzweigten, gesättigten Alkohols mit 3 bis 6 Kohlenstoffatomen führt zu einer leichten Entfernbarkeit des Alkanols und dessen Zersetzungsprodukten und somit zur Erzielung eines niedrigen Gehalts an organischem Kohlenstoff im getemperten Niederschlag. Im Gegensatz dazu sind beispielsweise Reduktionsmittel wie etwa Benzylalkohol beziehungsweise dessen Zersetzungsprodukte aus der Reduktion wesentlich schlechter zu entfernen, was letztlich einen nachteilig hohen Gehalt an organischem Kohlenstoff bedingt.

Ferner können beim erfindungsgemäßen Verfahren noch weitere reduzierend wirkende Komponenten eingesetzt werden. Als Beispiele seien Ethanol, Ameisensäure und Oxalsäure genannt. Bevorzugt wird das erfindungsgemäße Verfahren ohne Zugabe weiterer, reduzierend wirkender Komponenten durchgeführt.

Das Vanadiumpentoxid wird bevorzugt in Form eines Pulvers, besonders bevorzugt in einem Kornbereich von 50

bis 500 μm eingesetzt. Liegen deutlich größere Partikel vor, so wird der Feststoff vor dessen Einsatz zerkleinert und gegebenenfalls gesiebt. Geeignete Apparate sind beispielsweise Kugelmühlen oder Planetenmühlen.

Des Weiteren können bei der Herstelltung des Katalysator-Precursors sogenannte Promotor-Komponenten zugegeben werden. Als geeignete Promotoren sind die Elemente der 1. bis 15. Gruppe des Periodensystems sowie deren Verbindungen genannt. Geeignete Promotoren sind beispielsweise in WO 97/12674 und WO 95/26817 sowie in US 5,137,860, US 5,296,436, US 5,158,923 und US 4,795,818 beschrieben. Bevorzugt werden als Promotoren Verbindungen der Elemente Kobald, Molybdän, Eisen, Zink, Hafnium, Zirkon, Lithium, Titan, Chrom, Mangan, Nickel, Kupfer, Bor, Silicium, Antimon, Zinn, Niob und Wismut, besonders bevorzugt Molybdän, Eisen, Zink, Antimon, Wismut, Lithium. Die promotierten Katalysatoren können einen oder mehrere Promotoren enthalten. Im Allgemeinen werden die Promotor-Komponenten während des Schrittes (a), d.h. der genannten Umsetzung des Vanadiumpentoxids in Gegenwart eines primären oder sekundären, nichtcyclischen oder cyclischen, unverzweigten oder verzweigten, gesättigten Alkohols mit 3 bis 6 Kohlenstoffatomen mit 102 bis 110 %iger Phosphorsäure zugegeben. Der Gehalt an Promotoren beträgt in Summe im fertigen Katalysator im Allgemeinen nicht mehr als etwa 5 Gew.-%, jeweils als Oxid gerechnet.

Werden nach dem erfindungsgemäßen Verfahren promotierte Katalysator-Precursor hergestellt, so wird der Promotor im Allgemeinen beim Zusammenbringen des Vanadiumpentoxids, der 102 bis 110 %ige Phosphorsäure und des primären oder sekundären, nichtcyclischen oder cyclischen, unverzweigten oder verzweigten, gesättigten Alkohols mit 3 bis 6 Kohlenstoffatomen in Form eines anorganischen oder organischen Salzes zugegeben. Geeignete Promotor-Verbindungen sind beispielsweise die Acetate, Acetylacetonate, Oxalate, Oxide oder Alkoxide der zuvor genannten Promotormetalle, wie etwa Cobalt(II)-acetat, Cobalt(II)-acetylacetonat, Cobalt(II)-chlorid, Molybdän (VI)-oxid, Molybdän(III)-chlorid, Eisen(III)-acetylacetonat, Eisen(III)-chlorid, Zink(II)-oxid, Zink(II)-acetylacetonat, Lithiumchlorid, Lithiumoxid, Bismut (III) -chlorid, Bismut(III)-ethylhexanoat, Nickel (II)-ethylhexanoat, Nickel (II)-oxalat, Zirkonylchlorid, Zirkon(IV)-butoxid, Silizium(IV)-ethoxid, Niob(V)-chlorid und Niob(V)-oxid. Für weitere Details sei auf die zuvor genannten WO-Offenlegungsschriften und US-Patente verwiesen.

Das Zusammenbringen des Vanadiumpentoxids, der 102 bis 110 %ige Phosphorsäure und des primären oder sekundären, nichtcyclischen oder cyclischen, unverzweigten oder verzweigten, gesättigten Alkohols mit 3 bis 6 Kohlenstoffatomen kann beim erfindungsgemäßen Verfahren auf unterschiedliche Art und Weise erfolgen. So können die genannten Komponenten beispielsweise unverdünnt, verdünnt oder im Falle von Vanadiumpentoxid aufgeschlämmt vorgelegt oder zugegeben werden. Die Verdünnung beziehungsweise Aufschlämmung erfolgt im Allgemeinen mit dem primären oder sekundären, nichtcyclischen oder cyclischen, unverzweigten oder verzweigten, gesättigten Alkohol mit 3 bis 6 Kohlenstoffatomen.

Im Allgemeinen führt man das Zusammenbringen in dem für die anschließende Umsetzung geeigneten Reaktionsapparat, beispielsweise einem Rührkessel, unter Mischung durch. Die zusammenzubringenden Komponenten in unverdünnter, verdünnter oder aufgeschlämmter Form sind in der Regel auf eine Temperatur im Bereich von 0 bis 160°C temperiert, wobei die zusammenzubringenden Komponenten natürlich eine unterschiedliche Temperatur besitzen können.

Ohne limitierend zu wirken seien im Folgenden einige Varianten des Zusammenbringens beschrieben.

In einer Variante des Zusammenbringens führt man die Komponenten bei einer Temperatur im Bereich von 0 bis 50°C im Reaktionsapparat unter Rühren zusammen. Dabei ist die Reihenfolge der Zufuhr der einzelnen Komponenten im Allgemeinen unwesentlich.

In einer anderen Variante des Zusammenbringens legt man eine Aufschlämmung von Vanadiumpentoxid in einem primären oder sekundären, nichtcyclischen oder cyclischen, unverzweigten oder verzweigten, gesättigten Alkohol mit 3 bis 6 Kohlenstoffatomen im Reaktionsapparat vor und bringt diese dann bevorzugt auf eine Temperatur von 50 bis 160°C und besonders bevorzugt von 50 bis 100°C. In diese Aufschlämmung führt man anschließend die 102 bis 110 %ige Phosphorsäure, welche gegebenenfalls mit einem primären oder sekundären, nichtcyclischen oder cyclischen, unverzweigten oder verzweigten, gesättigten Alkohol mit 3 bis 6 Kohlenstoffatomen verdünnt sein kann, unter Rühren zu. Zur Herabsetzung der Viskosität der zuzuführenden Phosphorsäure ist es gegebenenfalls vorteilhaft, diese auf eine Temperatur im Bereich von 40 bis 100°C zu temperieren.

In einer weiteren Variante des Zusammenbringens legt man, wie bei der zuletztgenannten Variante bereits beschrieben, eine Aufschlämmung von Vanadiumpentoxid in einem primären oder sekundären, nichtcyclischen oder cyclischen, unverzweigten oder verzweigten, gesättigten Alkohol mit 3 bis 6 Kohlenstoffatomen im Reaktionsapparat vor und erhitzt diese unter Rühren ebenfalls auf eine Temperatur von 50 bis 160°C. Im Unterschied zur letztgenannten Variante liegt der bevorzugte Temperaturbereich aber mit 80 bis 160°C höher. Des Weiteren beläßt man das System über einen Zeitraum von etwa 0,5 bis mehreren, beispielsweise bis zu 10 Stunden unter Rückflußbedingungen, um eine Reduktion des Vanadiumpentoxids zu bewirken. Anschließend gibt man dann die 102 bis 110 %ige Phosphorsäure, welche gegebenenfalls mit einem primären oder sekundären, nichtcyclischen oder cyclischen, unverzweigten oder verzweigten, gesättigten Alkohol mit 3 bis 6 Kohlenstoffatomen verdünnt sein kann, unter weiterem Rühren zu.

In einer vierten Variante des Zusammenbringens legt man die 102 bis 110 %ige Phosphorsäure, welche gegebenenfalls mit einem primären oder sekundären, nichtcyclischen oder cyclischen, unverzweigten oder verzweigten, gesättigten Alkohol mit 3 bis 6 Kohlenstoffatomen verdünnt sein kann, vor und bringt diese dann bevorzugt auf eine Temperatur von 50 bis 160°C und besonders bevorzugt von 50 bis 100°C. Anschließend führt man Vanadiumpentoxid als Feststoff oder gegebenenfalls in Form einer Aufschlämmung in einem primären oder sekundären, nichtcyclischen oder cyclischen, unverzweigten oder verzweigten, gesättigten Alkohol mit 3 bis 6 Kohlenstoffatomen zu. Das Vanadiumpentoxid beziehungsweise dessen Aufschlämmung kann gegebenenfalls ebenfalls auf eine erhöhte Temperatur, beispielsweise 50 bis 100°C temperiert sein.

In einer fünften Variante des Zusammenbringens legt man den primären oder sekundären, nichtcyclischen oder cyclischen, unverzweigten oder verzweigten, gesättigten Alkohol mit 3 bis 6 Kohlenstoffatomen vor und bringt diesen dann bevorzugt auf eine Temperatur von 50 bis 160°C und besonders bevorzugt von 50 bis 100°C. Anschließend führt man Vanadiumpentoxid als Feststoff oder gegebenenfalls in Form einer Aufschlämmung in einem primären oder sekundären, nichtcyclischen oder cyclischen, unverzweigten oder verzweigten, gesättigten Alkohol mit 3 bis 6 Kohlenstoffatomen sowie die 102 bis 110 %ige Phosphorsäure, welche gegebenenfalls mit einem primären oder sekundären, nichtcyclischen oder cyclischen, unverzweigten oder verzweigten, gesättigten Alkohol mit 3 bis 6 Kohlenstoffatomen verdünnt sein kann, unter Rühren zu. Das Vanadiumpentoxid beziehungsweise dessen Aufschlämmung kann gegebenenfalls ebenfalls auf eine erhöhte Temperatur, beispielsweise 50 bis 100°C temperiert sein. Zur Herabsetzung der Viskosität der zuzuführenden Phosphorsäure ist es gegebenenfalls vorteilhaft, diese auf eine Temperatur im Bereich von 40 bis 100°C zu temperieren. Mit der Zugabe des Vanadiumpentoxids und der 102 bis 110 %igen Phosphorsäure kann zeitgleich oder auch zeitversetzt begonnen werden. Bevorzugt beginnt man mit der Zugabe von Vanadiumpentoxid und führt die 102 bis 110 %igen Phosphorsäure erst im weiteren Verlauf der Vanadiumpentoxid-Zugabe oder auch erst im Anschluß daran zu.

Das relative molare Verhältnis der 102 bis 110 %ige Phosphorsäure zum Vandiumpentoxid wird im Allgemeinen entsprechend dem gewünschten Verhältnis im Katalysator-Precursor eingestellt. Bevorzugt beträgt das molare Phosphor/Vanadium-Verhältnis im Reaktionsgemisch zur Herstellung des Katalysator-Precursors 1,0 bis 1,5 und besonders bevorzugt 1,1 bis 1,3.

Die Menge des primären oder sekundären, nichtcyclischen oder cyclischen, unverzweigten oder verzweigten, gesättigten Alkohols mit 3 bis 6 Kohlenstoffatomen sollte vorteilhafterweise über der für die Reduktion des Vanadiums von der Oxidationstufe +5 auf eine Oxidationstufe im Bereich +3,5 bis +4,5 stöchiometrisch erforderlichen Menge liegen. Die Menge sollte ferner so zu bemessen sein, dass mit dem Vanadiumpentoxid eine Aufschlämmung gebildet werden kann, welche eine intensive Vermischung mit der 102 bis 110 %ige Phosphorsäure ermöglicht. Im Allgemeinen beträgt das molare Verhältnis des Alkohols zum Vanadiumpentoxid 10 bis 25 und bevorzugt 12 bis 20.

Sind die Komponenten Vanadiumpentoxid, die 102 bis 110 %ige Phosphorsäure und der primäre oder sekundäre, nichtcyclische oder cyclische, unverzweigte oder verzweigte, gesättigte Alkohol mit 3 bis 6 Kohlenstoffatomen zusammengefügt, so wird das Gemisch zur Umsetzung der genannten Verbindungen über einem Zeitraum von üblicherweise mehreren Stunden auf eine Temperatur von 80 bis 160°C erhitzt. Der zu wählende Temperaturbereich ist von verschiedenen Faktoren, beispielsweise dem Siedepunkt des zugegebenen Alkohols abhängig und durch einfache Versuche zu optimieren. Beim Einsatz des ganz besonders bevorzugten Isobutanols erhitzt man bevorzugt auf eine Temperatur von 90 bis 120°C und besonders bevorzugt von 100 bis 110°C. Die flüchtige Verbindungen, wie beispielsweise Wasser, der Alkohol und dessen Abbauprodukte, wie etwa Aldehyd oder Carbonsäure, verdampfen aus der Reaktionsmischung und können entweder abgeführt oder partiell oder vollständig kondensiert und rückgeführt werden. Bevorzugt ist die partielle oder vollständige Rückführung durch Erhitzen unter Rückfluß. Besonders bevorzugt ist die vollständige Rückführung. Die Umsetzung bei erhöhter Temperatur beansprucht im Allgemeinen mehrere Stunden und ist von vielen Faktoren, wie beispielsweise der Art der zugegebenen Komponenten oder der Temperatur abhängig. Zudem können auch über die Temperatur und die gewählte Erhitzungsdauer in einem gewissen Bereich die Eigenschaften des Katalysator-Precursor eingestellt und beeinflußt werden. Die Parameter Temperatur und Zeit können für ein bestehendes System durch wenige Versuche einfach optimiert werden. Üblicherweise liegt der Zeitbedarf für die genannte Umsetzung bei 1 bis 25 Stunden.

Schritt (b)

Nach Beendigung der Umsetzung wird der gebildete Niederschlag isoliert, wobei vor der Isolierung gegebenenfalls noch eine Abkühlphase sowie eine Lagerungs- oder Alterungsphase der abgekühlten Reaktionsmischung zwischengeschaltet werden können. Bei der Isolierung wird der Niederschlag von der flüssigen Phase abgetrennt. Geeignete Methoden sind beispielsweise Filtrieren, Dekantieren oder Zentrifugieren. Bevorzugt wird der Niederschlag durch Filtrieren oder Zentrifugieren isoliert. Die Isolierung des Niederschlags erfolgt im Allgemeinen ebenfalls in einem Temperaturbereich von 0 bis 160°C, wobei Temperaturen im Bereich von 50 bis 150°C, insbesondere von 80 bis 150°C bevorzugt sind.

Der isolierte Niederschlag kann ungewaschen oder gewaschen weiterverarbeitet werden. Das Waschen des isolierten Niederschlags hat den Vorteil, dass noch anhaftende Reste des Alkanols sowie dessen Abbauprodukte weiter verringert werden können. Als geeignete Lösungsmittel für den Waschvorgang seien beispielsweise Alkohole (z.B. Methanol, Ethanol, 1-Propanol, 2-Propanol sowie der bei der vorhergehenden Umsetzung gewählte primäre oder sekundäre, nichtcyclische oder cyclische, unverzweigte oder verzweigte, gesättigte Alkohol mit 3 bis 6 Kohlenstoffatomen), aliphatische und/oder aromatische Kohlenwasserstoffe (z.B. Pentan, Hexan, Benzine, Benzol, Toluol, Xylole), Ketone (z.B. 2-Propanon (Aceton), 2-Butanon, 3-Pentanon, Ether (z.B. 1,2-Dimethoxyethan, Tetrahydrofuran, 1,4-Dioxan) oder deren Mischungen genannt. Wird der isolierte Niederschlag gewaschen, so wird bevorzugt der bei der vorhergehenden Umsetzung gewählte primäre oder sekundäre, nichtcyclische oder cyclische, unverzweigte oder verzweigte, gesättigte Alkohol mit 3 bis 6 Kohlenstoffatomen eingesetzt.

Der isolierte Niederschlag kann feucht oder getrocknet weiterverarbeitet werden. Im Allgemeinen wird der isolierte Niederschlag getrocknet. Die Trocknung kann unter verschiedenen Bedingungen durchgeführt werden. Im Allgemeinen führt man sie bei einem Druck von 0,0 ("Vakuum") bis 0,1 MPa abs ("Atmosphärendruck") durch. Die Trocknungstemperatur liegt in der Regel bei 30 bis 250°C, wobei bei einer Trocknung unter Vakuum gegenüber einer Trocknung unter Atmosphärendruck vielfach niedrigere Temperaturen angewandt werden können. Die gegebenenfalls bei der Trocknung überstehende Gasatmosphäre kann Sauerstoff, Wasserdampf und/oder Inertgase, wie etwa Stickstoff, Kohlendioxid oder Edelgase enthalten. Bevorzugt führt man die Trocknung bei einem Druck von 1 bis 30 kPa abs und einer Temperatur von 50 bis 200°C unter sauerstoffhaltiger oder sauerstofffreier Restgasatmosphäre, wie beispielsweise Luft oder Stickstoff, durch.

Die Trocknung kann beispielsweise in der Filtriereinrichtung selbst oder in einem separaten Apparat, zum Beispiel einem Trokkenschrank oder einem kontinuierlich arbeitenden Bandtrockner durchgeführt werden.

Schritt (c)

Bei Schritt (c) wird im isolierten Niederschlag durch Temperung in einem Temperaturbereich von 250 bis 350°C ein Gehalt an organischem Kohlenstoff von ≤1,1 Gew.-% einstellt, wobei das getemperte Produkt nach Zusatz von 3,0 Gew.-% Graphit als inneren Standard unter Anwendung von CuKα-Strahlung ($\lambda$ = 1,54 $10^{-10}$ m) ein Pulver-Röntgenbeugungsdiagramm ergibt, das im $2\theta$-Bereich ein Peakhöhenverhältnis des Peaks einer eventuell vorhandenen Pyrophosphatphase bei 28,5° zu dem vom Graphit stammenden Peak bei 26,6° von ≤ 0, 1 aufweist.

Als organischer Kohlenstoff ist jener Kohlenstoff zu verstehen, welcher aus der pulverförmigen Probe durch Versetzen mit 10 Gew.-%iger wässriger Salzsäure und anschließendem Erwärmen unter Durchleitung eines Stickstoffstroms nicht ausgetrieben werden kann. Der Gehalt an organischem Kohlenstoff errechnet sich aus der Differenz zwischen dem Gesamt-Kohlenstoffgehalt und dem Gehalt an anorganischem Kohlenstoff.

Zur Bestimmung des Gesamt-Kohlenstoffgehalts wird eine genau eingewogene pulverförmige Probe in Gegenwart eines reinen Sauerstoff-Stroms in ein auf etwa 1000°C erhitztes Quarzrohr gegeben, geglüht und das im Verbrennungsgas enthaltene Kohlendioxid quantitativ bestimmt. Aus der Menge an detektiertem Kohlendioxid und der Probeneinwaage kann dann der Gesamt-Kohlenstoffgehalt rückgerechnet werden. Eine genaue Methodenbeschreibung befindet sich in den Beispielen unter "Bestimmung des Gesamt-Kohlenstoffgehalts".

Zur Bestimmung des Gehalts an anorganischem Kohlenstoff wird eine genau eingewogene pulverförmige Probe mit 10 Gew.-%iger wässriger Salzsäure versetzt, das freigesetzte Kohlendioxid unter langsamen Erwärmen unter Durchleitung eines Stickstoffstroms ausgetrieben und das ausgetriebene Kohlendioxid quantitativ bestimmt. Aus der Menge an detektiertem Kohlendioxid und der Probeneinwaage kann dann der Gehalt an anorganischem Kohlenstoff rückgerechnet werden. Eine genaue Methodenbeschreibung befindet sich in den Beispielen unter "Bestimmung des Gehalts an anorganischem Kohlenstoff".

Beim erfindungsgemäßen Verfahren wird durch die Temperung bei Schritt (c) ein Gehalt an organischem Kohlenstoff von bevorzugt ≤ 1,0 Gew.-%, besonders bevorzugt ≤ 0,8 Gew.-% und ganz besonders bevorzugt ≤ 0,7 Gew.-% eingestellt.

Der Gesamt-Kohlenstoffgehalt beträgt im Allgemeinen ≤ 2,0 Gew.-%, bevorzugt ≤1,5 Gew.-% und besonders bevorzugt ≤1,2 Gew.-%.

Durch die erfindungsgemäße Maßnahme der Einstellung eines niedrigen Gehalts an organischem Kohlenstoff von ≤1,1 Gew.-% wird bei der späteren Kalzinierung des partikulären Katalysator-Precursors eine Schädigung des Katalysators minimiert beziehungsweise vermieden sowie die Einstellung einer sehr gleichmäßigen Oxidationsstufe des Vanadiums über den gesamten Katalysatorformkörper hinweg ermöglicht. Bei höheren Gehalten an organischem Kohlenstoff, d.h. bei Gehalten oberhalb 1,1 Gew.-% besteht die Gefahr der nachhaltigen mechanischen und/oder chemischen Schädigung durch einen lokal stark erhöhten Druck infolge Verflüchtigung, Zersetzung und/oder Oxidation der organischen Verbindungen, durch lokale Heißstellen infolge einer chemischen Reaktionen der organischen Verbindungen mit dem Katalysatormaterial und/oder der Gasphase (z.B. Oxidation durch Sauerstoff) und durch lokale Reduktion des Katalysatormaterials infolge einer chemischen Reaktionen zwischen den reduzierend wirkenden organischen Verbindungen und dem Katalysatormaterial. Die zuletzt genannte lokale Reduktion des Katalysatormaterials bei Gehalten an organischen Kohlenstoff oberhalb 1,1 Gew.-% führt zu einer sehr ungleichmäßigen Oxidationsstufe des Vanadiums über den Katalysatorformkörper, wobei die innenliegenden Bereiche eine deutlich niedrigere Oxidationsstufe des Vanadiums aufweisen als die außen liegenden Bereich.

Wie bereits erwähnt, erfolgt die Temperung in Schritt (c) derart, dass das getemperte Produkt nach Zusatz von 3,0 Gew.-% Graphit als inneren Standard unter Anwendung von CuKα-Strahlung (λ = 1,54 · 10⁻¹⁰ m) ein Pulver-Röntgenbeugungsdiagramm ergibt, das im 2θ-Bereich ein Peakhöhenverhältnis des Peaks einer eventuell vorhandenen Pyrophosphatphase bei 28,5° zu dem vom Graphit stammenden Peak bei 26, 6° von ≤ 0,1 aufweist.

Das Röntgenbeugungsdiagramm gibt die Intensität der gebeugten Röntgenstrahlung (in counts per second = cps) als Funktion des zweifachen Beugungswinkels 2θ wieder. Zur Aufnahme des Pulver-Röntgenbeugungsdiagramms wird der pulverförmige und mit 3 Gew.-% Graphit innig vermischte Niederschlag eingesetzt. Die Aufnahme des Pulver-Röntgenbeugungsdiagramms erfolgt mit einem sogenannten Pulverdiffraktometer mit variabler Apertur- und Streustrahlblende, wobei in Reflexion gemessen wird. Die jeweilige Peakhöhe ergibt sich aus der Differenz zwischen der maximalen Intensität des jeweiligen Signals und dem ermittelten Untergrund. Eine genaue Methodenbeschreibung befindet sich in den Beispielen unter "Röntgendiffraktometrische Analyse des getemperten Niederschlags".

Beim erfindungsgemäßen Verfahren erfolgt die Temperung in Schritt (c) derart, dass das getemperte Produkt nach Zusatz von 3,0 Gew.-% Graphit als inneren Standard unter Anwendung von CuK-α-Strahlung (λ = 1,54 ·10⁻¹⁰ m) ein Pulver-Röntgenbeugungsdiagramm ergibt, das im 2θ-Bereich ein Peakhöhenverhältnis des Peaks einer eventuell vorhandenen Pyrophosphatphase bei 28,5° zu dem vom Graphit stammenden Peak bei 26,6° von bevorzugt ≤0,08, besonders bevorzugt ≤0,05 und ganz besonders Bevorzugt ≤0,02 aufweist.

Beim erfindungsgemäßen Verfahren erfolgt die Temperung in Schritt (c) in einem Temperaturbereich von 250 bis 350°C, bevorzugt von 260 bis 350°C und besonders bevorzugt von 270 bis 340°C. Die Temperung kann prinzipiell in einem weiten Druckbereich durchgeführt werden, wobei die Anwendung niedriger Drücke die Entfernung organischer Komponenten in der Regel fördert. Im Allgemeinen führt man die Temperung bei einem Druck von 0,0 ("Vakuum") bis 0,15 MPa abs, bevorzugt bei etwa 0,1 MPa abs ("Atmosphärendruck") durch.

Die Temperung beansprucht im Allgemeinen mehrere Minuten bis mehrere Stunden und ist von vielen Faktoren, wie beispielsweise der Konzentration der eingesetzten Phosphorsäure, der Art des eingesetzten primären oder sekundären, nichtcyclischen oder cyclischen, unverzweigten oder verzweigten, gesättigten Alkohols mit 3 bis 6 Kohlenstoffatomen, der weiteren Behandlung des abgeschiedenen Niederschlags (z.B. Alterung des Niederschlags) sowie der gewählten Temper-Temperatur abhängig. So ist es beispielsweise möglich, dass eine zeitlich lange Temperung bei niedriger Temperatur zu einem ähnlichen Ergebnis führt wie eine zeitlich kürzere Temperung bei einer mittleren oder höheren Temperatur. Die Parameter Temperatur und Zeit können für ein bestehendes System durch wenige Versuche einfach optimiert werden. In der Regel liegt die erforderliche Temperzeit bei 0,5 bis 10 Stunden.

Die bei der Temperung vorhandene Gasatmosphäre kann Sauerstoff, Wasserdampf und/oder Inertgase, wie etwa Stickstoff, Kohlendioxid oder Edelgase enthalten. Bevorzugt erfolgt die Temperung unter Luft.

Die Temperung kann beispielsweise diskontinuierlich oder kontinuierlich durchgeführt werden. Als geeignete Apparate seien Trockenschrank, Muffelofen, Bandkalziniervorrichtung, Wirbeltrockner oder Drehrohr genannt. Um ein gleichmäßig getempertes Produkt zu erhalten, ist es in der Regel vorteilhaft ein kontinuierlich arbeitendes Temperverfahren mit Vermischung des zu tempernden Pulvers einzusetzen. Besonders bevorzugt ist daher die Temperung in einem kontinuierlich betriebenen Drehrohr.

Schritt (d)

In Schritt (d) wird das aus Schritt (c) erhaltene, getemperte Produkt zu Partikeln mit einem gemittelten Durchmesser von mindestens 2 mm und bevorzugt von mindestens 3 mm geformt. Unter dem gemittelten Durchmesser eines Partikels ist der Mittelwert aus der kleinsten und der größten Abmessung zwischen zwei planparallelen Platten zu verstehen.

Unter Partikeln sind sowohl regellos geformte Partikel als auch geometrisch geformte Partikel, sogenannte Formkörper, zu verstehen. Bevorzugt wird das aus Schritt (c) erhaltene, getemperte Produkt zu Formkörper geformt. Als geeignete Formkörper seien beispielsweise Tabletten, Zylinder, Hohlzylinder, Kugeln, Stränge, Wagenräder oder Extrudate genannt. Besondere Formen, wie beispielsweise "Trilobes" und "Tristars" (siehe WO 93/01155) oder Formkörper mit mindestens einer Einkerbung an der Außenseite (siehe US 5,168,090) sind ebenfalls möglich.

Erfolgt die Formgebung des getemperten Produkts durch Tablettierung, wie es beispielsweise bei der Herstellung von Tabletten, Zylindern und Hohlzylindern üblich ist, so wird dem Pulver im Allgemeinen ein Tablettierhilfsmittel zugesetzt und innig vermischt. Tablettierhilfsmittel sind in der Regel katalytisch inert und verbessern die Tablettiereigenschaften des sogenannten Pulvers, beispielsweise durch Erhöhung der Gleit- und Rieselfähigkeit. Als geeignetes und bevorzugtes Tablettierhilfsmittel sei Graphit genannt. Die zugesetzten Tablettierhilfsmittel verbleiben in der Regel im aktivierten Katalysator. Typischerweise liegt der Gehalt an Tablettierhilfsmittel im fertigen Katalysator bei etwa 2 bis 6 Gew.-%.

Besonders bevorzugt werden Formkörper mit einer im Wesentlichen hohlzylinderförmigen Struktur geformt. Unter einer im Wesentlichen hohlzylinderförmigen Struktur ist eine Struktur zu verstehen, welche im Wesentlichen einen Zylinder mit einer zwischen den beiden Deckelflächen hindurchgehenden Öffnung umfaßt. Der Zylinder ist charakterisiert durch zwei im Wesentlichen parallele Dekkelflächen und einer Mantelfläche, wobei der Querschnitt des Zylinders, d.h.

parallel zu den Deckelflächen, im Wesentlichen von kreisförmiger Struktur ist. Der Querschnitt der hindurchgehenden Öffnung, d.h. parallel zu den Deckelflächen des Zylinders, ist im Wesentlichen ebenfalls von kreisförmiger Struktur. Bevorzugt befindet sich die hindurchgehende Öffnung mittig zu den Deckelflächen, wobei andere räumliche Anordnungen damit nicht ausgeschlossen sind.

Der Begriff "im Wesentlichen" weißt darauf hin, daß Abweichungen von der Idealgeometrie, wie beispielsweise leichte Deformationen der kreisförmigen Struktur, nicht planparallel ausgerichtete Dekkelflächen, abgeplatzte Ecken und Kanten, Oberflächenrauhigkeit oder Einkerbungen in der Mantelfläche, den Deckelflächen oder der Innenfläche der hindurchgehenden Bohrung beim erfindungsgemäßen Katalysator mit umfaßt sind. Im Rahmen der Genauigkeit der Tablettierkunst sind kreisförmige Deckelflächen, ein kreisförmiger Querschnitt der hindurchgehenden Bohrung, parallel ausgerichtete Deckelflächen und makroskopisch glatte Oberflächen bevorzugt.

Die im Wesentlichen hohlzylinderförmige Struktur kann beschrieben werden durch einen äußeren Durchmesser $d_1$, einer Höhe h als Abstand der beiden Deckelflächen und einem Durchmesser des inneren Lochs (hindurchgehende Öffnung) $d_2$. Der äußere Durchmesser $d_1$ beträgt bevorzugt 3 bis 10 mm, besonders bevorzugt 4 bis 8 mm, ganz besonders bevorzugt 4,5 bis 6 mm. Die Höhe h beträgt bevorzugt 1 bis 10 mm, besonders bevorzugt 2 bis 6 mm, ganz besonders bevorzugt 2 bis 3,5 mm. Der Durchmesser der hindurchgehenden Öffnung $d_2$ beträgt bevorzugt 1 bis 8 mm, besonders bevorzugt 2 bis 6 mm, ganz besonders bevorzugt 2 bis 3 mm. Besonders bevorzugt ist eine hohlzylinderförmige Struktur, welche (a) ein Verhältnis der Höhe h zum Durchmesser der hindurchgehenden Öffnung $d_2$ von höchstens 1,5 und (b) ein Verhältnis der geometrischen Oberfläche $A_{geo}$ zum geometrischen Volumen $V_{geo}$ von mindestens 2 $mm^{-1}$ aufweist, wie sie etwa in WO 01/68245 beschrieben ist.

Durch die weitgehende bis vollständige Vermeidung der Ausbildung der Pyrophosphatphase im getemperten Niederschlag in Schritt (c) und der Formgebung in Schritt (d) vor der eigentlichen Kalzinierung, d.h. der Überführung der Vanadylhydrogenphosphat-Hemihydrat-Phase ($VOHPO_4 \cdot \frac{1}{2}H_2O$) in die katalytisch aktive Pyrophosphatphase (($VO)_2P_2O_7$) unter Abspaltung von Wasser, wird überraschenderweise eine für die katalytischen Eigenschaften vorteilhaftere Katalysatorstruktur erhalten als bei einer Formgebung nach der genannten Phasenumwandlung.

In einer besonders bevorzugten Ausführungsform zur Herstellung des Katalysator-Precursors wird in einem geeigneten Rührapparat eine Aufschlämmung von Vanadiumpentoxid in Isobutanol vorgelegt, auf eine Temperatur im Bereich von 50 bis 100°C erwärmt und unter weiterem Rühren 102 bis 110 %ige Phosphorsäure zugeführt. Anschließend wird für mehrere Stunden unter weiterem Rühren auf eine Temperatur im Bereich von 100 bis 110°C unter Rückfluß erhitzt. Im Anschluß daran wird die heiße Suspension filtriert, mit wenig Isobutanol gewaschen und bei einer Temperatur im Bereich von 100 bis 200°C unter Vakuum getrocknet. Der isolierte und getrocknete Niederschlag wird nun kontinuierlich, bevorzugt in einem Drehrohr, unter Luft bei etwa Atmosphärendruck in einem Temperaturbereich von 250 bis 350°C und einer mittleren Verweilzeit im Bereich von 0,5 bis 5 Stunden, bevorzugt von 1 bis 3 Stunden, getempert. Die Temperbedingungen wurden dabei derart gewählt, dass das getemperte Produkt einen Gehalt an organischen Kohlenstoff von ≤1,1 Gew.-% besitzt und nach Zusatz von 3,0 Gew.-% Graphit als inneren Standard unter Anwendung von $CuK\alpha$-Strahlung ($\lambda = 1,54 \cdot 10^{-10}$ m) ein Pulver-Röntgenbeugungsdiagramm ergibt, das im $2\theta$-Bereich ein Peakhöhenverhältnis des Peaks einer eventuell vorhandenen Pyrophosphatphase bei 28,5° zu dem vom Graphit stammenden Peak bei 26,6° von ≤0,1 aufweist. Das erhaltene, getemperte Produkt wird nun mit 2 bis 6 Gew.-% Graphit innig vermischt und zu tablettenförmigen oder hohlzylinderförmigen Formkörpern tablettiert. Bevorzugt wird der Katalysator-Precursor zu Hohlzylindern mit einem äußeren Durchmesser $d_1$ von 4,5 bis 6 mm, einer Höhe h von 2 bis 3,5 mm und einem Durchmesser der hindurchgehenden Öffnung $d_2$ von 2 bis 3 mm tablettiert.

Des Weiteren wurde ein Katalysator-Precursor für die Herstellung von Maleinsäureanhydrid durch heterogenkatalytische Gasphasenoxidation eines Kohlenwasserstoffs mit mindestens vier Kohlenstoffatomen gefunden, welcher gemäß dem oben beschriebenen erfindungsgemäßen Verfahren erhältlich ist.

Das erfindungsgemäße Verfahren ermöglicht die Herstellung eines Vanadium, Phosphor und Sauerstoff enthaltenden Katalysator-Precursors für die Herstellung von Maleinsäureanhydrid durch heterogenkatalytische Gasphasenoxidation eines Kohlenwasserstoffs mit mindestens vier Kohlenstoffatomen, wobei die Herstellung des Katalysator-Precursors technisch einfach durchzuführen ist und der Katalysator-Precursor einen besonders niedrigen Gehalt an organischem Kohlenstoff aufweist und als selektivitäts- und aktivitätsbestimmende Vorstufe die Herstellung eines Katalysators mit einer hohen Aktivität und einer hohen Selektivität ermöglicht.

Weiterhin ist Gegenstand der Erfindung, ein Verfahren zur Herstellung eines Vanadium, Phosphor und Sauerstoff enthaltenden Katalysators für die Herstellung von Maleinsäureanhydrid durch heterogenkatalytische Gasphasenoxidation eines Kohlenwasserstoffs mit mindestens vier Kohlenstoffatomen, durch Behandlung eines Vanadium, Phosphor- und Sauerstoff enthaltenden Katalysator-Precursors in mindestens einer Atmosphäre, umfassend Sauerstoff ($O_2$), Wasserstoffoxid ($H_2O$) und/oder Inertgas in einem Temperaturbereich von 250 bis 600°C, das dadurch gekennzeichnet ist, dass man als Katalysator-Precursor einen erfindungsgemäßen Katalysator-Precursor gemäß obiger Beschreibung einsetzt.

Als geeignete Inertgase seien beispielsweise Stickstoff, Kohlendioxid und Edelgase genannt.

Die Kalzinierung kann diskontinuierlich, beispielsweise in einem Schachtofen, Hordenofen, Muffelofen oder Wärmeschrank oder kontinuierlich, beispielsweise in einem Drehrohr, Bandkalzinierofen oder Drehkugelofen durchgeführt werden. Sie kann sukzessive verschiedene Abschnitte hinsichtlich der Temperatur wie Aufheizen, Konstanthalten der Temperatur oder Abkühlen und sukzessive verschiedene Abschnitte hinsichtlich der Atmosphären wie beispielsweise sauerstoffhaltige, wasserdampfhaltige, sauerstofffreie Gasatmosphären enthalten. Geeignete Präformierungsverfahren sind beispielsweise in den Patenten US 5,137,860 und US 4,933,312 und der Offenlegungsschrift WO 95/29006 beschrieben, auf die ausdrücklich jedoch nicht limitierend bezug genommen wird. Besonders bevorzugt ist die kontinuierliche Kalzinierung in einem Bandkalzinierofen mit mindestens zwei, beispielsweise zwei bis zehn Kalzinierungszonen, welche gegebenenfalls eine unterschiedliche Gasatmosphäre und eine unterschiedliche Temperatur aufweisen. Durch geeignete, an das jeweilige Katalysatorsystem angepaßte Kombination von Temperaturen, Behandlungsdauern und Gasatmosphären kann die mechanische und katalytische Eigenschaft des Katalysators beeinflußt und somit gezielt eingestellt werden.

Beim erfindungsgemäßen Verfahren bevorzugt ist eine Kalzinierung, bei der man den Katalysatorprecursor

(i) in mindestens einer Kalzinierungszone in einer oxidierenden Atmosphäre mit einem Sauerstoff-Gehalt von 2 bis 21 Vol.-% auf eine Temperatur von 200 bis 350°C aufheizt und unter diesen Bedingungen bis zur Einstellung der gewünschten mittleren Oxidationsstufe des Vanadiums beläßt; und

(ii) in mindestens einer weiteren Kalzinierungszone in einer nicht-oxidierenden Atmosphäre mit einem Sauerstoff-Gehalt von $\leq$ 0,5 Vol.-% und einem Wasserstoffoxid-Gehalt von 20 bis 75 Vol.-% auf eine Temperatur von 300 bis 500°C aufheizt und $\geq$0,5 Stunden unter diesen Bedingungen beläßt.

Bei Schritt (i) wird der Katalysatorprecursor in einer oxidierend wirkenden Atmosphäre mit einem Gehalt an molekularem Sauerstoff von im Allgemeinen 2 bis 21 Vol.-% und bevorzugt von 5 bis 21 Vol.-% bei einer Temperatur von 200 bis 350°C und bevorzugt von 250 bis 350°C über einen Zeitraum, der wirksam ist, die gewünschte mittlere Oxidationsstufe des Vanadiums einzustellen, belassen. Im Allgemeinen setzt man bei Schritt (i) Mischungen aus Sauerstoff, Inertgasen (z.B. Stickstoff oder Argon), Wasserstoffoxid (Wasserdampf) und/oder Luft sowie Luft ein. Aus der Sicht des durch die Kalzinierungszone(n) geführten Katalysatorprecursors kann die Temperatur während des Kalzinierschrittes (i) konstant gehalten werden, im Mittel steigen oder fallen. Da dem Schritt (i) im Allgemeinen eine Aufheizphase vorangeschaltet ist, wird die Temperatur in der Regel zunächst ansteigen, um dann bei dem gewünschten Endwert einzupendeln. Im Allgemeinen ist daher der Kalzinierungszone von Schritt (i) mindestens eine weitere Kalzinierungszone zur Aufheizung des Katalysatorprecursors vorangeschaltet.

Der Zeitraum, über den die Temperung in Schritt (i) aufrecht erhalten wird, ist beim erfindungsgemäßen Verfahren bevorzugt derart zu wählen, dass sich eine mittlere Oxidationsstufe des Vanadiums auf einen Wert von +3,9 bis +4,4, bevorzugt von +4,0 bis +4,3, einstellt. Die Bestimmung der mittleren Oxidationsstufe des Vanadiums erfolgt über potentiometrische Titration nach nach der in den Beispielen beschriebenen Methode.

Da die Bestimmung der mittleren Oxidationsstufe des Vanadiums während der Kalzinierung aus apparativen und zeitlichen Gründen nur äußerst schwierig zu bestimmen ist, ist der erforderliche Zeitraum vorteilhafterweise in Vorversuchen experimentell zu bestimmen. In der Regel dient hierzu eine Meßreihe, bei der unter definierten Bedingungen getempert wird, wobei die Proben nach unterschiedlichen Zeiten aus dem System entfernt, abgekühlt und bezüglich der mittleren Oxidationsstufe des Vanadiums analysiert werden.

Der bei Schritt (i) erforderliche Zeitraum ist im Allgemeinen abhängig von der Natur des Katalysatorprecursors, der eingestellten Temperatur und der gewählten Gasatmosphäre, insbesondere des Sauerstoff-Gehalts. Im Allgemeinen erstreckt sich der Zeitraum bei Schritt (i) auf eine Dauer von über 0,5 Stunden und bevorzugt von über 1 Stunde. Im Allgemeinen ist ein Zeitraum von bis zu 4 Stunden, bevorzugt von bis zu 2 Stunde zur Einstellung der gewünschten mittleren Oxidationsstufe ausreichend. Unter entsprechend eingestellten Bedingungen (z.B. unterer Bereich des Temperaturintervalls und/oder geringer Gehalt an molekularem Sauerstoff) kann aber auch ein Zeitraum von über 6 Stunden erforderlich sein.

Bei Schritt (ii) wird die erhaltene Katalysatorzwischenstufe in einer nicht-oxidierenden Atmosphäre mit einem Gehalt an molekularem Sauerstoff von $\leq$0,5 Vol.-% und an Wasserstoffoxid (Wasserdampf) von 20 bis 75 Vol.-%, bevorzugt von 30 bis 60 Vol.-% bei einer Temperatur von 300 bis 500°C und bevorzugt von 350 bis 450°C über einen Zeitraum von $\geq$0,5 Stunden, bevorzugt 2 bis 10 Stunden und besonders bevorzugt 2 bis 4 Stunden belassen. Die nicht-oxidierende Atmosphäre enthält neben dem genannten Wasserstoffoxid im Allgemeinen überwiegend Stickstoff und/oder Edelgase, wie beispielsweise Argon, wobei hierunter keine Einschränkung zu verstehen ist. Auch Gase, wie beispielsweise Kohlendioxid sind prinzipiell geeignet. Bevorzugt enthält die nicht-oxidierende Atmosphäre $\geq$40 Vol.-% Stickstoff. Aus der Sicht des durch die Kalzinierungszone(n) geführten Katalysatorprecursors kann die Temperatur während des Kalzinierschrittes (ii) konstant gehalten werden, im Mittel steigen oder fallen. Wird Schritt (ii) bei einer höheren oder tieferen Temperatur als Schritt (i) durchgeführt, so befindet sich zwischen den Schritten (i) und (ii) in der Regel eine Aufheiz- oder Abkühlphase, welche gegebenenfalls in einer weiteren Kalzinierungszone implementiert ist. Um eine verbesserte Trennung zur sauerstoffhaltigen Atmosphäre des Schrittes (i) zu ermöglichen, kann diese weitere Kalzinierungszone zwischen (i) und (ii) beispielsweise zur Spülung mit Inertgas, wie beispielsweise Stickstoff, gespült werden. Bevorzugt wird Schritt (ii) bei einer um 50 bis 150°C höheren Temperatur als Schritt (i) durchgeführt.

Im Allgemeinen umfasst die Kalzinierung einen weiteren, zeitlich nach Schritt (ii) durchzuführenden Schritt (iii), bei dem man den kalzinierten Katalysatorprecursor in einer Inertgas-Atmosphäre auf eine Temperatur von ≤300°C, bevorzugt von ≤ 200°C und besonders bevorzugt von ≤ 150°C abkühlt.

Vor, zwischen und/oder nach den Schritten (i) und (ii), beziehungsweise (i), (ii) und (iii) sind bei der Kalzinierung nach dem erfindungsgemäßen Verfahren weitere Schritte möglich. Ohne limitierend zu wirken seien als weitere Schritte beispielsweise Änderungen in der Temperatur (Aufheizen, Abkühlen), Änderungen in der Gasatmosphäre (Umstellung der Gasatmosphäre), weitere Haltezeiten, Überführungen der Katalysatorzwischenstufe in andere Apparate oder Unterbrechungen des gesamten Kalziniervorgangs genannt.

Da der Katalysator-Precursor in der Regel vor Beginn der Kalzinierung eine Temperatur von < 100°C besitzt, ist dieser vor Schritt (i) üblicherweise aufzuheizen. Das Aufheizen kann unter Anwendung verschiedener Gasatmosphären durchgeführt werden. Vorzugsweise wird das Aufheizen in einer oxidierend wirkenden Atmosphäre, wie unter Schritt (i) definiert, oder einer Inertgas-Atmosphäre, wie unter Schritt (iii) definiert, durchgeführt. Auch ein Wechsel der Gasatmosphäre während der Aufheizphase ist möglich. Besonders bevorzugt ist das Aufheizen in der oxidierend wirkenden Atmosphäre, welche auch in Schritt (i) angewendet wird.

Des Weiteren wurde ein Katalysator für die Herstellung von Maleinsäureanhydrid durch heterogenkatalytische Gasphasenoxidation eines Kohlenwasserstoffs mit mindestens vier Kohlenstoffatomen gefunden, welcher gemäß dem oben beschriebenen erfindungsgemäßen Verfahren erhältlich ist.

Der durch das erfindungsgemäße Verfahren bevorzugt hergestellte Katalysator weist ein Phosphor/Vanadium-Atomverhältnis von 0,9 bis 1,5, besonders bevorzugt von 0,9 bis 1,2 und ganz besonders bevorzugt von 1,0 bis 1,1, eine mittlere Oxidationsstufe des Vanadiums von +3,9 bis +4,4 und besonders bevorzugt von 4,0 bis 4,3, eine BET-Oberfläche von 10 bis 50 $m^2$/g und besonders bevorzugt von 20 bis 40 $m^2$/g, ein Porenvolumen von 0,1 bis 0,5 ml/g und besonders bevorzugt von 0,2 bis 0,4 ml/g und eine Schüttdichte von 0,5 bis 1,5 kg/l und besonders bevorzugt 0,5 bis 1,0 kg/l auf.

Der durch Kalzinierung des erfindungsgemäßen Katalysator-Precursors erhältliche Katalysator zeichnet sich durch eine weitgehend homogene Oxidationsstufe des Vanadiums innerhalb der einzelnen Katalysator-Partikel sowie zwischen den verschiedenen Katalysator-Partikeln untereinander aus. Der erfindungsgemäße Katalysator ermöglicht in der heterogenkatalytische Gasphasenoxidation eines Kohlenwasserstoffs mit mindestens vier Kohlenstoffatomen zu Maleinsäureanhydrid eine hohe Kohlenwasserstoffbelastung bei gleichzeitig hohem Umsatz, eine hohe Aktivität, eine hohe Selektivität sowie eine hohe Raum/Zeit-Ausbeute.

Weiterhin ist Gegenstand der Erfindung ein Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen, das dadurch gekennzeichnet ist, dass man einen erfindungsgemäßen Katalysator gemäß obiger Beschreibung einsetzt.

Beim erfindungsgemäßen Verfahren zur Herstellung von Maleinsäureanhydrid werden im Allgemeinen Rohrbündelreaktoren eingesetzt. Als Kohlenwasserstoffe werden im Allgemeinen aliphatische und aromatische, gesättigte und ungesättigte Kohlenwasserstoffe mit mindestens vier Kohlenstoffatomen, wie beispielsweise 1,3-Butadien, 1-Buten, 2-cis-Buten, 2-trans-Buten, n-Butan, $C_4$-Gemisch, 1,3-Pentadien, 1,4-Pentadien, 1-Penten, 2-cis-Penten, 2-trans-Penten, n-Pentan, Cyclopentadien, Dicyclopentadien, Cyclopenten, Cyclopentan, $C_5$-Gemisch, Hexene, Hexane, Cyclohexan und Benzol geeignet. Bevorzugt eingesetzt werden 1-Buten, 2-cis-Buten, 2-trans-Buten, n-Butan, Benzol oder deren Mischungen geeignet. Besonders bevorzugt ist der Einsatz von n-Butan und n-Butan-haltigen Gasen und Flüssigkeiten. Das verwendete n-Butan kann beispielsweise aus dem Erdgas, aus Steamcrackern oder FCC-Crackern stammen.

Die Zugabe des Kohlenwasserstoffs erfolgt im Allgemeinen mengengeregelt, d.h. unter stetiger Vorgabe einer definierten Menge pro Zeiteinheit. Der Kohlenwasserstoff kann in flüssiger oder gasförmiger Form dosiert werden. Bevorzugt ist die Dosierung in flüssiger Form mit anschließender Verdampfung vor Eintritt in den Rohrbündelreaktor.

Als Oxidationsmittel werden Sauerstoff enthaltende Gase, wie beispielsweise Luft, synthetische Luft, ein mit Sauerstoff angereichertes Gas oder auch sogenannter "reiner", d.h. z.B. aus der Luftzerlegung stammender Sauerstoff eingesetzt. Auch das Sauerstoff-enthaltende Gas wird mengengeregelt zugegeben.

Das durch den Rohrbündelreaktor zu leitende Gas enthält im Allgemeinen eine Kohlenwasserstoff-Konzentration von 0,5 bis 15 Vol.-% und eine Sauerstoff-Konzentration von 8 bis 25 Vol.-%. Der zu einhundert Vol.-% fehlende Anteil setzt sich aus weiteren Gasen wie beispielsweise Stickstoff, Edelgasen, Kohlenmonoxid, Kohlendioxid, Wasserdampf, oxygenierte Kohlenwasserstoffe (z.B. Methanol, Formaldehyd, Ameisensäure, Ethanol, Acetyaldehyd, Essigsäure, Propanol, Propionaldehyd, Propionsäure, Acrolein, Crotonaldehyd) und deren Mischungen zusammen. Bevorzugt beträgt der n-Butan-Anteil an der Gesamtmenge an Kohlenwasserstoff ≥90 % und besonders bevorzugt ≥95 %.

Zur Gewährung einer langen Katalysatorstandzeit und weiterer Erhöhung von Umsatz, Selektivität, Ausbeute, Katalysator-Belastung und Raum/Zeit-Ausbeute wird dem Gas beim erfindungsgemäßen Verfahren bevorzugt eine flüchtige Phosphorverbindung zugeführt. Ihre Konzentration beträgt zu Beginn, d.h. am Reaktoreingang, mindestens 0,2 Volumen-ppm, d.h. $0,2 \cdot 10^{-6}$ Volumenanteile der flüchtigen Phosphorverbindungen bezogen auf das Gesamtvolumen des Gases am Reaktoreingang. Bevorzugt ist ein Gehalt von 0,2 bis 20 Volumen-ppm, besonders bevorzugt von 0,5 bis 10 Volumen-ppm. Als flüchtige Phosphorverbindungen sind all jene Phosphor-enthaltende Verbindungen zu verstehen, welche in der gewünschten Konzentration unter den Einsatzbedingungen gasförmig vorliegen. Als geeignete flüchtige Phosphorverbindungen sind beispielsweise Phosphine und Phosphorsäureester genannt. Besonders bevorzugt sind die $C_1$- bis $C_4$-Alkyl-Phosphorsäureester, ganz besonders bevorzugt Trimethylphosphat, Triethylphosphat und Tripropylphosphat, insbesondere Triethylphosphat.

Das erfindungsgemäße Verfahren wird im Allgemeinen bei einer Temperatur von 350 bis 480°C durchgeführt. Unter der genannten Temperatur wird die Temperatur der im Rorbündelreaktor befindlichen Katalysatorschüttung verstanden, welche bei Ausübung des Verfahrens in Abwesenheit einer chemischen Reaktion vorliegen würde. Ist diese Temperatur nicht an allen Stellen exakt gleich, so meint der Begriff den Zahlenmittelwert der Temperaturen längs der Reaktionszone. Insbesondere bedeutet dies, daß die wahre, am Katalysator vorliegende Temperatur aufgrund der Exothermie der Oxidationsreaktion auch außerhalb des genannten Bereichs liegen kann. Bevorzugt wird das erfindungsgemäße Verfahren bei einer Temperatur von 380 bis 460°C, besonders bevorzugt 380 bis 430°C durchgeführt.

Das erfindungsgemäße Verfahren kann bei einem Druck unterhalb von Normaldruck (z.B. bis 0,05 MPa abs) als auch oberhalb von Normaldruck (z.B. bis 10 MPa abs) ausgeübt werden. Darunter ist der in der Rohrbündelreaktor-Einheit vorliegende Druck zu verstehen. Bevorzugt ist ein Druck von 0,1 bis 1,0 MPa abs, besonders bevorzugt 0,1 bis 0,5 MPa abs.

Das erfindungsgemäße Verfahren kann in zwei bevorzugten Verfahrensvarianten, der Variante mit "geradem Durchgang" und der Variante mit "Rückführung" durchgeführt werden. Beim "geraden Durchgang" wird aus dem Reaktoraustrag Maleinsäureanhydrid und gegebenenfalls oxygenierte Kohlenwasserstoff-Nebenprodukte entfernt und das verbleibende Gasgemisch ausgeschleust und gegebenenfalls thermisch verwertet. Bei der "Rückführung" wird aus dem Reaktoraustrag ebenfalls Maleinsäureanhydrid und gegebenenfalls oxygenierte Kohlenwasserstoff-Nebenprodukte entfernt, das verbleibende Gasgemisch, welches nicht-umgesetzten Kohlenwasserstoff enthält, ganz oder teilweise zum Reaktor rückgeführt. Eine weitere Variante der "Rückführung" ist die Entfernung des nicht-umgesetzten Kohlenwasserstoffs und dessen Rückführung zum Reaktor.

In einer besonders bevorzugten Ausführungsform zur Herstellung von Maleinsäureanhydrid setzt man n-Butan als Ausgangs-Khohlenwasserstoff ein und führt die heterogenkatalytische Gasphasenoxidation "geraden Durchgang" an dem erfindungsgemäßen Katalysator durch.

Das erfindungsgemäße Verfahren unter Verwendung der erfindungsgemäßen Katalysatoren ermöglicht eine hohe Kohlenwasserstoff-Belastung des Katalysators bei einem hohen Umsatz infolge einer hohen Aktivität. Das erfindungsgemäße Verfahren ermöglicht ferner eine hohe Selektivität, eine hohe Ausbeute und daher auch eine hohe Raum/Zeit-Ausbeute an Maleinsäureanhydrid.

Definitionen

Die in dieser Schrift verwendeten Größen sind, falls nicht anders erwähnt, wie folgt definiert:

$$\text{Raum/Zeit-Ausbeute} = \frac{m_{Maleinsäureanhydrid}}{V_{Katalysato} \cdot t}$$

$$\text{Belastung} = \frac{V_{Kohlenwasserstoff}}{V_{Katalysato} \cdot t}$$

$$\text{Umsatz U} = \frac{n_{KW,Reaktor,ein} - n_{KW,Reaktor,aus}}{n_{KW,Reaktor,ein}}$$

$$\text{Selektivität } S = \frac{n_{MSA Reaktor,aus}}{n_{KW,Reaktor,ein} - n_{KW,Reaktor,aus}}$$

$$\text{Ausbeute } A = U \cdot S$$

| | |
|---|---|
| $m_{Maleinsäureanhydrid}$ | Masse an produziertem Maleinsäureanhydrid [g] |
| $V_{Katalysator}$ | Schüttvolumen Katalysator, summiert über alle Reaktionszonen [1] |
| t | Zeiteinheit [h] |
| $V_{Kohlenwasserstoff}$ | auf 0°C und 0,1013 MPa normiertes Volumen des Kohlenwasserstoffs in der Gasphase [N1] (Rechnerische Größe. Liegt ein Kohlenwasserstoff unter diesen Bedingungen in der Flüssigphase vor, so wird über das ideale Gasgesetz das hypothetische Gasvolumen berechnet.) |
| U | Umsatz an Kohlenwasserstoffen pro Reaktordurchgang |
| S | Selektivität bzgl. Maleinsäureanhydrid pro Reaktordurchgang |
| A | Ausbeute an Maleinsäureanhydrid pro Reaktordurchgang |
| $n_{KW}$, Reaktor, ein | Stoffmengenstrom an Kohlenwasserstoffen am Reaktoreingang [mol/h] |
| $n_{KW}$, Reaktor, aus | Stoffmengenstrom an Kohlenwasserstoffen am Reaktorausgang [mol/h] |
| $n_{KW}$, Anlage, ein | Stoffmengenstrom an Kohlenwasserstoffen am Eingang der Anlage [mol/h] |
| $n_{KW}$, Anlage, aus | Stoffmengenstrom an Kohlenwasserstoffen am Ausgang der Anlage [mol/h] |
| $n_{MSA}$, Reaktor, aus | Stoffmengenstrom an Maleinsäureanhydrid am Reaktorausgang [mol/h] |
| $n_{MSA}$, Anlage, aus | Stoffmengenstrom an Maleinsäureanhydrid am Ausgang der Anlage [mol/h] |

Beispiele

Bestimmung des Rest-Isobutanolgehalts im getrockneten Katalysator-Precursor

Zur Bestimmung des Rest-Isobutanolgehalts wurden etwa 4 g des getrockneten pulverförmigen Katalysator-Precursors und etwa 10 g N,N-Dimethylformamid in eine beheizbare Rührapparatur mit Rückflußkühler genau eingewogen. Anschließend wurde unter Rühren auf Siedetemperatur aufgeheizt und 30 Minuten unter diesen Bedingungen belassen. Nach der Abkühlung wurde die Suspension filtriert und im Filtrat der Gehalt an Isobutanol durch Gaschromatographie quantitativ erfasst. Der Rest-Isobutanolgehalt wurde dann aus der ermittelten Konzentration an Isobutanol im N,N-Dimethylformamid und der eingewogenen Mengen an N,N-Dimethylformamid und Katalysator-Precursor berechnet.

Bestimmung des Gesamt-Kohlenstoffgehalts

Zur Bestimmung des Gesamt-Kohlenstoffgehalts wurden etwa 50 bis 200 mg der genau eingewogenen pulverförmigen Probe in Gegenwart eines reinen Sauerstoff-Stroms in ein auf etwa 1000°C erhitztes Quarzrohr gegeben und geglüht. Das erhaltene Verbrennungsgas wurde durch eine IR-Zelle geleitet und der Gehalt an Kohlendioxid quantitativ bestimmt. Aus der Menge an detektiertem Kohlendioxid konnte der Gesamt-Kohlenstoffgehalt der Probe rückgerechnet werden.

Bestimmung des Gehalts an anorganischem Kohlenstoff

Zur Bestimmung des Gehalts an anorganischem Kohlenstoff wurden etwa 50 bis 200 mg der genau eingewogenen pulverförmigen Probe mit 10 Gew.-%iger wässriger Salzsäure versetzt. Das freigesetzte Kohlendioxid wurde unter langsamen Erwärmen unter Durchleitung eines Stickstoffstroms ausgetrieben und zur Reinigung durch eine Kaskade, umfassend eine mit Isopropanol/Trockeneis gekühlte Kühlfalle, zwei Absorptionsgefäße mit Kaliumpermanganatlösung, ein Absorptionsgefäß mit konzentrierter Schwefelsäure und ein Braunstein-Röhrchen, geleitet. Der gereingte Gasstrom wurde in eine sogenante Coulometerzelle geleitet, welche mit einer Lösung von 0,1 Gew.-% Thymolphthalein in Dimethylsulfoxid gefüllt war und die Änderung des Farbumschlages photometrisch vermessen. Aus der Änderung der Transmission kann die eingeleitete Menge an Kohlendioxid und somit der Gehalt an anorganischem Kohlenstoff rückgerechnet werden.

Bestimmung des Gehalts an organischem Kohlenstoff

Der Gehalt an organischem Kohlenstoff errechnet sich aus der Differenz zwischen dem Gesamt-Kohlenstoffgehalt und dem Gehalt an anorganischem Kohlenstoff.

Röntgendiffraktometrische Analyse des getemperten Niederschlags

Zur röntgendiffraktometrischen Analyse wurde der pulverförmige und mit 3 Gew.-% Graphit innig vermischte Niederschlag in einem Röntgenpulverdiffraktometer vom Typ "D5000 Fa. Siemens Theta/ Theta" vermessen. Die Meßparameter waren wie folgt:

| | |
|---|---|
| Kreisdurchmesser | 435 mm |
| Röntgenstrahlung | CuK$\alpha$ ($\lambda$ =1,54 $\cdot 10^{-10}$m) |
| Röhrenspannung | 40 kV |
| Röhrenstrom | 30 mA |
| Aperturblende | variabel V20 |
| Streustrahlblende | variabel V20 |
| Sekundärmonochromator | Graphit |
| Monochromatorblende | 0,1 mm |
| Szintillationszähler | |
| Detektorblende | 0,6 mm |
| Schrittweite | 0,02° 2$\theta$ |
| Schrittmodus | kontinuierlich |
| Meßzeit | 2,4 s / Schritt |
| Meßgeschwindigkeit | 0,5° 2$\theta$ / min |

Die jeweilige Peakhöhe ergibt sich aus der Differenz zwischen der maximalen Intensität des jeweiligen Signals und dem ermittelten Untergrund.

Bestimmung der Seitendruckfestigkeit der Hohlzylinder

Zur Bestimmung der Seitendruckfestigkeit wurden in nacheinander folgenden Messungen die Hohlzylinder mit der gerundeten Seitenfläche jeweils auf die plane Metall-Auflageplatte einer entsprechenden Meßeinrichtung gestellt. Die beiden planparallelen Dekkelflächen befanden sich somit in vertikaler Richtung. Nun wurde ein planer Metall-Stempel von oben mit einer Vorschubgeschwindigkeit von 1,6 mm/min auf den Hohlzylinder zugefahren und der zeitliche Verlauf der Krafteinwirkung auf den Hohlzylinder bis zu dessen Bruch aufgezeichnet. Die Seitendruckfestigkeit des einzelnen Hohlzylinders entspricht der maximal eingewirkten Kraft.
Zur Bestimmung der Seitendruckfestigkeit wurden unter Mittelwertbildung jeweils 30 Einzelmessungen durchgeführt.

Bestimmung der mittleren Oxidationsstufe des Vanadiums

Die Bestimmung der mittleren Oxidationsstufe des Vanadiums erfolgte über potentiometrische Titration.
Zur Bestimmung wurden jeweils 200 bis 300 mg der Probe unter Argonatmosphäre in eine Mischung aus 15 mL 50 %iger Schwefelsäure und 5 mL 85 %iger Phosphorsäure gegeben und unter Erwärmen gelöst. Die Lösung wurde anschließend in ein Titrationsgefäß, welches mit zwei Pt-Elektroden ausgestattet ist, überführt. Die Titrationen wurden jeweils bei 80°C durchgeführt. Zuerst erfolgte eine Titration mit 0,1 molarer Kaliumpermanganat-Lösung. Wurden zwei Stufen in der potentiometrischen Kurve erhalten, so lag das Vanadium in einer mittleren Oxidationsstufe von +3 bis kleiner +4 vor. Wurde nur eine Stufe erhalten, so lag das Vanadium in einer Oxidationsstufe von +4 bis kleiner +5 vor.

Bei dem erstgenannten Fall (zwei Stufen / $+3 \leq V_{ox} < +4$) enthielt die Lösung kein $V^{5+}$, das heißt das gesamte Vanadium wurde titrimetrisch erfaßt. Über den Verbrauch der 0,1 molaren Kaliumpermanganat-Lösung und der Lage der beiden Stufen wurde die Menge an $V^{3+}$ und $V^{4+}$ berechnet. Der gewichtete Mittelwert ergab dann die mittlere Oxidationsstufe.

Bei dem zweitgenannten Fall (eine Stufe / $+4 \leq V_{ox} < +5$) konnte aus dem Verbrauch der 0,1 molaren Kaliumpermanganat-Lösung die Menge an $V^{4+}$ berechnet werden. Durch anschließende Reduktion des gesamten $V^{5+}$ der erhaltenen Lösung mit einer 0,1 molaren Ammonium-eisen(II)-sulfat-Lösung und erneute Oxidation mit 0,1 molarer Kaliumpermanganat-Lösung konnte die Gesamtmenge an Vanadium berechnet werden. Die Differenz zwischen der Gesamtmenge an Vanadium und der Menge an $V^{4+}$ ergab die ursprünglich vorhandene Menge an $V^{5+}$. Der gewichtete Mittelwert ergab dann die mittlere Oxidationsstufe.

Versuchsanlage

Die Versuchsanlage war ausgestattet mit einer Zufuhr-Einheit und einem Reaktorrohr. Der Ersatz eines Rohrbündelreaktors durch ein Reaktorrohr ist im Labor- oder Technikumsmaßstab sehr gut möglich, sofern die Abmessungen des Reaktorrohres im Bereich eines technischen Reaktorrohres liegen. Die Anlage wurde im "geraden Durchgang" betrieben.

Der Kohlenwasserstoff wurde mengengeregelt in flüssiger Form über eine Pumpe zugegeben. Als Sauerstoffhaltiges Gas wurde Luft mengengeregelt zugegeben. Triethylphosphat (TEP) wurde ebenfalls mengengeregelt, gelöst in Wasser, in flüssiger Form zugegeben.

Die Rohrbündelreaktor-Einheit bestand aus einem Rohrbündelreaktor mit einem Reaktorrohr. Die Länge des Reaktorrohrs betrug 6,5 m, der Innendurchmesser 22,3 mm. Innerhalb des Reaktorrohres befand sich in einem Schutzrohr mit 6 mm Außendurchmesser ein Multi-Thermoelement mit 20 Temperaturmeßstellen. Das Reaktorrohr war von einem temperierbaren Wärmeträger-Kreislauf umgeben und wurde von dem Reaktionsgasgemisch von oben nach unten durchströmt. Die oberen 0,3 m des Reaktorrohres waren mit Inertmaterial gefüllt und bildeten die Vorheizzone. Die Reaktionszone enthielt jeweils 2,2 L Katalysator. Als Wärmeträgermedium wurde eine Salzschmelze eingesetzt.

Direkt nach der Rohrbündelreaktor-Einheit wurde gasförmiges Produkt entnommen und der gaschromatographischen on-line Analytik zugeführt. Der Hauptstrom des gasförmigen Reaktoraustrags wurde aus der Anlage ausgeschleust.

Die Anlage wurde wie folgt betrieben:

| | |
|---|---|
| Konzentration an n-Butan am Reaktoreingang = | 2,0 Vol.-% |
| GHSV = | 2000 Nl/ $l_{Katalysator} \cdot h$ |
| Druck am Reaktorausgang = | 0,2 MPa abs |
| Konzentration an Triethylphosphat (TEP) = | 2 Volumen-ppm |
| Konzentration an Wasserdampf = | 1,5 Vol.-% |

Beispiel 1: Herstellung des getrockneten Katalysator-Precursors im technischen Maßstab

In einem mit Stickstoff inertisierten, über Druckwasser außenbeheizbaren 8 m$^3$-Stahl/Email-Rührkessel mit Strombrechern wurden 6,1 m$^3$ Isobutanol vorgelegt. Nach Inbetriebnahme des dreistufigen Impellerrührers wurde das Isobutanol unter Rückfluß auf 90°C aufgeheizt. Bei dieser Temperatur wurde nun über die Förderschnecke mit der Zugabe von 736 kg Vanadiumpentoxid begonnen. Nachdem nach ca. 20 Minuten etwa 2/3 der gewünschten Menge an Vanadiumpentoxid zugegeben wurden, wurde bei weiterer Zugabe an Vanadiumpentoxid mit der Einpumpung von 900 kg 105 %iger Phosphorsäure begonnen. Zur Reinigung der Pumpe wurden weitere 0,2 m$^3$ Isobutanol nachgepumpt. Anschließend wurde das Reaktionsgemisch unter Rückfluß auf etwa 100 bis 108°C erhitzt und unter diesen Bedingungen 14 Stunden belassen. Im Anschluß daran wurde die heiße Suspension in eine zuvor mit Stickstoff inertisierte und beheizte Druckfilternutsche abgelassen und bei einer Temperatur von etwa 100°C bei einem Druck oberhalb der Filternutsche von bis zu 0,35 MPa abs abfiltriert. Der Nutschkuchen wurde durch stetiges Einleiten von Stickstoff bei 100°C und unter Rühren mit einem mittig angeordneten, in der Höhe verstellbaren Rührer innerhalb von etwa einer Stunde trockengeblasen. Nach dem Trockenblasen wurde auf ca. 155°C aufgeheizt und auf einen Druck von 15 kPa abs (150 mbar abs) evakuiert. Die Trocknung wurde bis zu einem Rest-Isobutanolgehalt von < 2 Gew.-% im getrockneten Katalysator-Precursor durchgeführt.

Um die gewünschte Menge an Katalysatorprecursor von etwa 9 t zu erhalten, wurden mehrere Ansätze durchgeführt.

Beispiel 2: Temperung, Tablettierung und Kalzinierung des Katalysator-Precusors aus Beispiel 1 im Labormaßstab

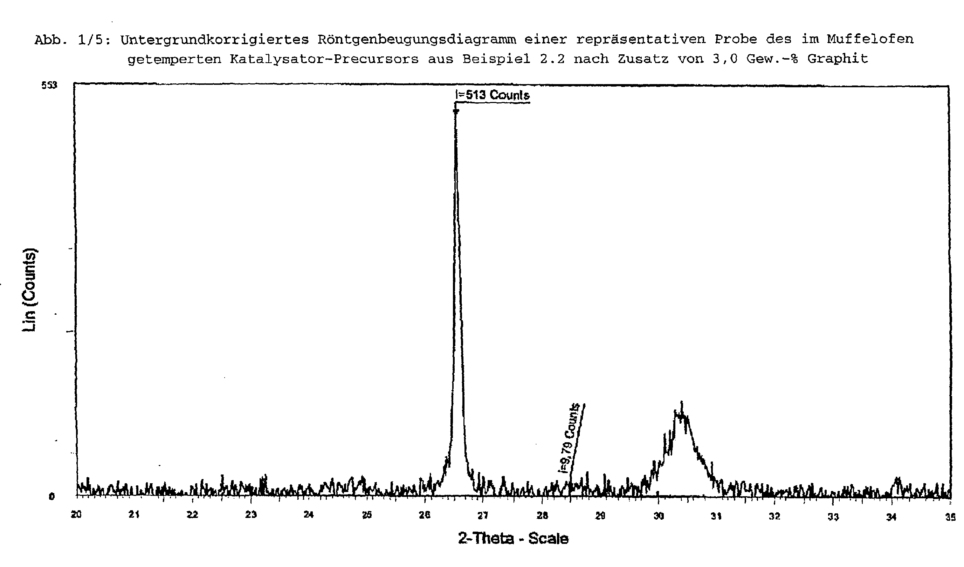

Von dem in Beispiel 1 erhaltenen, getrockneten Precursor-Pulver wurden etwa 10 kg Probe entnommen und in 20 Portionen von jeweils etwa 0,5 kg nacheinander in einem Muffelofen 5 Stunden unter Luft bei 250°C (10 Proben, Beispiel 2.1) beziehungsweise 300°C (10 Proben, Beispiel 2.2) getempert. Die bei derselben Temperatur getemperten Proben wurden anschließend innig vermischt und der Rest-Isobutanolgehalt, der Gesamt-Kohlenstoffgehalt, der Gehalt an anorganischem Kohlenstoff und der Gehalt an organischem Kohlenstoff bestimmt. Ferner wurde eine repräsentative Probe aus Beispiel 2.2 mit 3,0 Gew.-% Graphit vermischt und das Röntgenbeugungsdiagramm aufgenommen. Dieses ist in Abbildung 1 dargestellt. Die Ergebnisse der getemperten Katalysator-Precursor sind in Tabelle 1 dargestellt.

Tabelle 1: Analysendaten der im Muffelofen getemperten Katalysator-Precusor

|  | Beispiel 2.1* | Beispiel 2.2 |
|---|---|---|
| Temper-Temperatur | 250°C | 300°C |
| Rest-Isobutanolgehalt [Gew.-%] | 0 | 0 |
| Gesamt-Kohlenstoffgehalt [Gew.-%] | 1,5 | 0,77 |
| Gehalt an anorganischem Kohlenstoff [Gew.-%] | 0,15 | 0,35 |
| Gehalt an organischem Kohlenstoff [Gew.-%] | 1,35 | 0,42 |
| XRD-Peakhöhenverhältnis I(28,5°)/I(26,6°) | < 0,02 | 0,02 |
| * Vergleichsbeispiel | | |

Der erfindungsgemäße Katalysator-Precusor aus Beispiel 2.2 weist ein XRD-Peakhöhenverhältnis I(28,5°)/I (26,6°) von 0,02 und einen Gehalt an organischem Kohlenstoff von 0,42 Gew.-% auf. Der Katalysator-Precusor aus dem Vergleichsbeispiel 2.1 besitzt hingegen einen deutlich höheren Gehalt an organischen Kohlenstoff von 1,35 Gew.-%.

Die beiden getemperten Katalysator-Precursor wurden jeweils mit 3 Gew.-% Graphit vermischt und in einer Tablettiermaschine zu 5x3x2 mm Hohlzylindern (äußerer Durchmesser x Höhe x Durchmesser des inneren Lochs) mit einer Seitendruckfestigkeit von 20 N tablettiert. Je 4,5 kg der beiden Hohlzylinder-Proben wurden nacheinander in einen Umluftofen gegeben und wie folgt kalziniert:

Schritt (1): Erhitzen unter Luft auf 250°C mit einer Heizrate von 3°C/min.

Schritt (2): Weiteres Erhitzen unter Luft von 250 auf 350°C mit einer Heizrate von 2°C/min.

Schritt (3): Halten bei dieser Temperatur für 15 Minuten.

Schritt (4): Wechsel von der Luft-Atmosphäre auf eine Stickstoff/Wasserdampf-Atmosphäre (1:1) innerhalb von 20 Minuten.

Schritt (5): Erhitzen unter dieser Atmosphäre auf 425°C mit einer Heizrate von 1,7°C/min.

Schritt (6): Halten bei dieser Temperatur für 3 Stunden.

Schritt (7): Wechsel der Atmosphäre auf Stickstoff, und Abkühlung auf Raumtemperatur.

Die kalzinierten Hohlzylinder der nach Beispiel 2.1 und 2.2 hergestellten Katalysatoren wurden hinsichtlich der räumlichen Verteilung der mittleren Oxidationsstufe des Vanadiums untersucht. Hierzu wurden aus einer statistisch ausgewählten Menge der Hohlzylinder insgesamt etwa 200 bis 300 mg von der Oberfläche (d.h. vom äußeren Umfang, vom inneren Loch und den Deckelflächen) abgekratzt und daraus die mittlere Oxidationsstufe des Vanadiums im oberflächennahen Bereich bestimmt. Ferner wurden weitere 200 bis 300 mg aus den innenliegenden Bereich mechanisch isoliert und daraus die mittlere Oxidationsstufe des Vanadiums im innenliegenden Bereich bestimmt. Ferner wurde die BET-Oberfläche gemessen. Die erhaltenen Ergebnisse sind in Tabelle 2 dargestellt.

Tabelle 2: Mittlere Oxidationsstufe des Vanadiums und BET-Oberfläche der im Muffelofen unterschiedlich getemperten und anschließend unter identischen Bedingungen kalzinierten Proben

| | Beispiel 2.1* | Beispiel 2.2 |
|---|---|---|
| $V_{ox.}$ (oberflächennaher Bereich) | 4,3 | 4,17 |
| $V_{ox.}$ (innenliegender Bereich) | 3,97 | 4,17 |
| BET-Oberfläche [$m^2/g$] | 20 | 26 |
| * Vergleichsbeispiel | | |

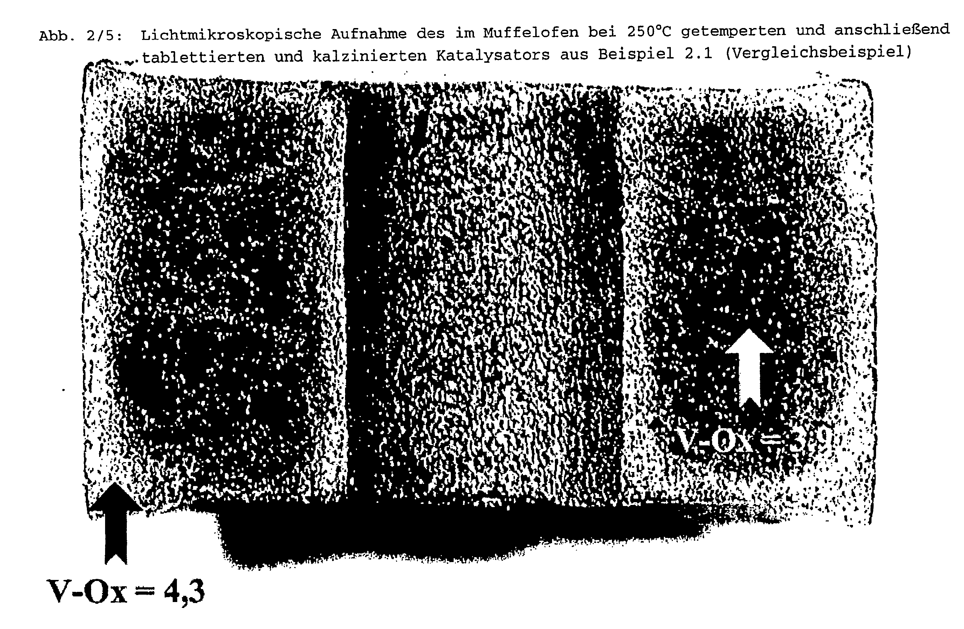

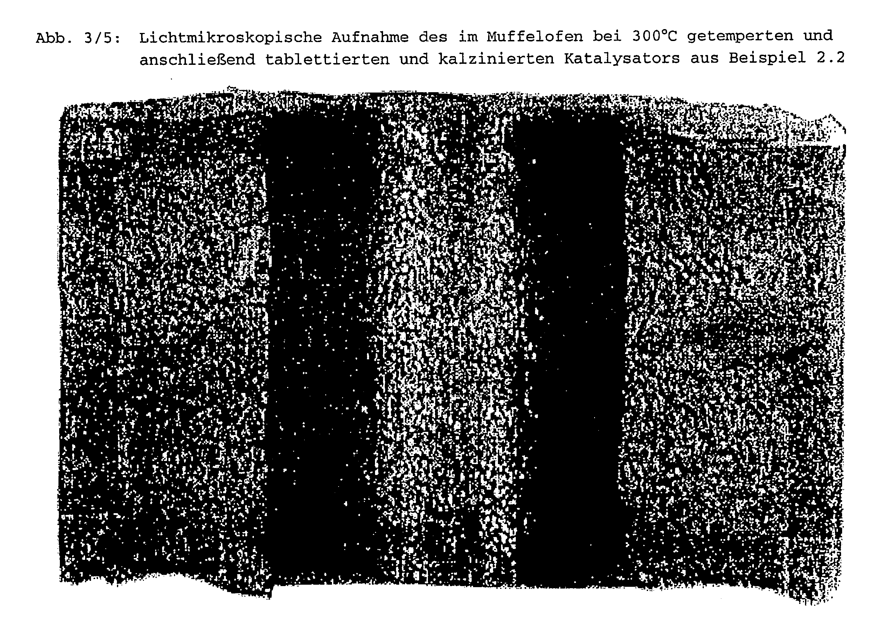

Wie aus Tabelle 2 hervorgeht, zeigt der Vergleichskatalysator aus Beispiel 2.1 einen signifikanten Unterschied zwischen der mittleren Oxidationsstufe des Vanadiums des oberflächennahen und des innenliegenden Bereichs. Im oberflächennahem Bereich beträgt diese 4,3 und im innenliegenden Bereich 3,97. Dieser signifikante Unterschied ist auch aus der in Abbildung 2 abgebildeten lichtmikroskopischen Aufnahme eines halbierten Hohlzylinders durch den hellen Oberflächenbereich und den dunklen inneren Bereich ersichtlich. Im Gegensatz weist der erfindungsgemäße Katalysator aus Beispiel 2.2 eine vollkommen gleichmäßig verteilte mittlere Oxidationsstufe des Vanadiums von 4,17 auf. Die in Abbildung 3 abgebildete lichtmikroskopische Aufnahme eines halbierten Hohlzylinders zeigt eine gleichmäßige Färbung innerhalb der beiden Bruchstellen.

Des Weiteren weist der erfindungsgemäße Katalysator aus Beispiel 2.2 mit 26 $m^2/g$ eine um etwa 30 rel.-% größere BET-Oberfläche auf als der Vergleichskatalysator aus Beispiel 2.1.

Beispiel 3: Katalytischer Test der Katalysatoren aus Beispiel 2

Mit jeweils 2,2 L einer statistischen Mischung der beiden im Umluftofen kalzinierten Katalysatoren aus Beispiel 2.1 und 2.2 wurden in der oben beschriebenen Versuchsanlage ein katalytischer Performancetest durchgeführt. Die Salzbadtemperatur wurde dabei derart eingestellt, dass ein n-Butan-Umsatz von etwa 84 % resultierte. Die erhaltenen Ergebnisse sind in Tabelle 3 wiedergegeben.

Tabelle 3: Ergebisse der katalytischen Tests

| | Katalysator nach Beispiel 2.1* (Temperung bei 250°C) | Katalysator nach Beispiel 2.2 (Temperung bei 300°C) |
|---|---|---|
| Salzbadtemperatur [°C] | 415 | 412 |
| Umsatz U [%] | 84,0 | 83,6 |
| Ausbeute A [%] | 50,7 | 55,7 |
| * Vergleichsbeispiel | | |

Der nach Beispiel 2.2 hergestellte, bei 300°C getemperte Katalysator zeigt bei nahezu gleichem Umsatz eine gegenüber dem nach Beispiel 2.1 hergestellten, bei 250°C getemperten Katalysator um 10 Relativ-% höhere Ausbeute an Maleinsäureanhydrid.

Beispiel 4: Temperung des Katalysator-Precusors aus Beispiel 1 im technischen Maßstab

Precursor-Pulver, das nach Beispiel 1 hergestellt wurde, wurde 2 Stunden unter Luft in einem Drehrohr mit einer Länge von 6,5 m, einem Innendurchmesser von 0,9 m und innenliegenden spiralförmigen Wendeln getempert.

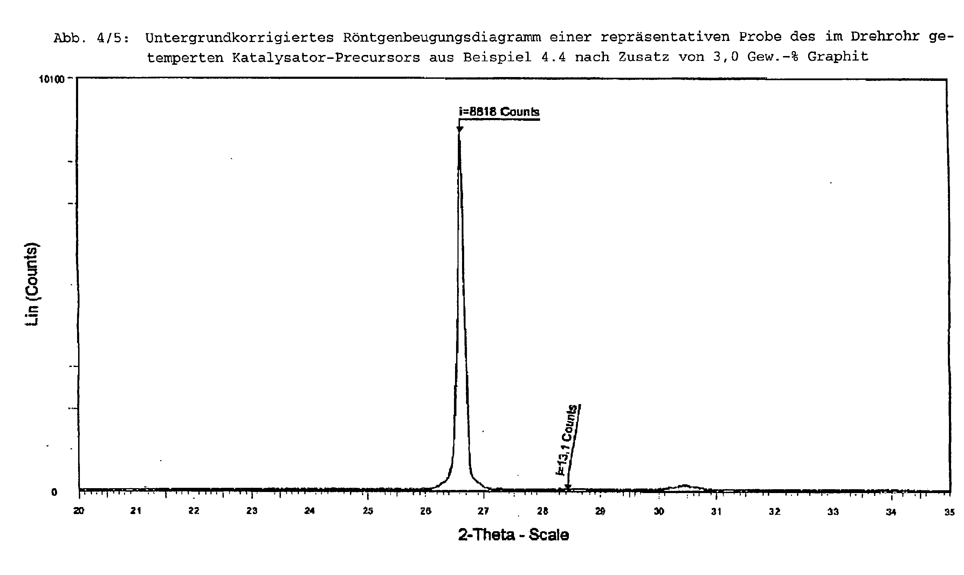

Die Drehzahl des Drehrohres betrug 0,4 U/min. Das Pulver wurde in einer Menge von 60 kg/h in das Drehrohr gefördert. Die Luftzufuhr betrug 100 m$^3$/h. Die direkt an der Außenseite des Drehrohrs gemessenen Temperaturen der fünf gleichlangen Heizzonen betrugen 250°C, 300°C, 340°C, 340°C und 340°C. Von dem im Drehrohr getemperten Katalysator-Precursor wurden über die gesamte Produktion hinweg in statistischen Abständen insgesamt 7 Proben entnommen und der Rest-Isobutanolgehalt, der Gesamt-Kohlenstoffgehalt, der Gehalt an anorganischem Kohlenstoff und der Gehalt an organischem Kohlenstoff bestimmt. Ferner wurde eine repräsentative Probe aus Beispiel 4.4 mit 3,0 Gew.-% Graphit vermischt und das Röntgenbeugungsdiagramm aufgenommen. Die Ergebnisse sind in Tabelle 4 und das Röntgenbeugungsdiagramm von Beispiel 4.4 in Abbildung 4 dargestellt.

Wie Tabelle 4 zeigt, liegen alle Analysendaten in einem sehr engen Bereich. Bei keiner der Proben konnte ein Restgehalt an Isobutanol nachgewiesen werden. Der Gesamt-Kohlenstoffgehalt lag in einem Bereich von 0,77 bis 1,10 Gew.-%, der Gehalt an anorganischem Kohlenstoff in einem Bereich von 0,34 bis 0,60 Gew.-% und der Gehalt an organischem Kohlenstoff in einem Bereich von 0,40 bis 0,67 Gew.-%. Das XRD-Peakhöhenverhältnis I(28,5°)/I(26,6°) des erfindungsgemäßen Katalysator-Precursors aus Beispiel 4.4 betrug < 0, 01.

Des Weiteren wurde die mittlere Oxidationsstufe des Vanadiums der Precursor-Proben bestimmt. Auch bei diesem Analysenwert lagen alle 7 Proben in einem sehr engen Bereich von 4,00 bis 4,04.

Beispiel 5: Tablettierung und Kalzinierung des Katalysator-Precursors aus Beispiel 4.5 und katalytischer Test

Etwa 4 kg des getemperten Katalysator-Precursors aus Beispiel 4.5 wurden wie in Beispiel 2 beschrieben mit Graphit vermischt, zu 5x3x2 mm Hohlzylindern (äußerer Durchmesser x Höhle x Durchmesser des inneren Lochs) tablettiert und in einem Umluftofen kalziniert. Mit 2,2 L davon wurden wie in Beispiel 3 beschrieben in der ober beschriebenen Versuchsanlage ein katalytischer Performancetest durchgeführt. Die Salzbadtemperatur wurde dabei derart eingestellt, dass ein n-Butan-Umsatz von etwa 84 % resultierte. Die erhaltenen Ergebnisse sind in Tabelle 5 wiedergegeben.

Tabelle 5: Ergebisse des katalytischen Tests

|  | Katalysator nach Beispiel 4.5 |
|---|---|
| Salzbadtemperatur [°C] | 410 |
| Umsatz U [%] | 84,0 |
| Ausbeute A [%] | 56,0 |

Bei einer Salzbadtemoperatur von 410°C und einem Umsatz von 84,0 % zeigte der nach Beispiel 4.5 getemperte Katalysator eine Ausbeute an Maleinsäureanhydrid von 56,0 %. Das erhaltene Ergebnis gleicht somit dem des nach Beispiel 2.2 getemperten Katalysators.

Beispiel 6: Nachstellung von "Examples 1-7" aus EP-A 0 056 183 (Vergleichsbeispiel)

In Beispiel 6 wurde "Examples 1-7" der EP-A 0 056 183 nachgestellt. Hierzu wurden 91 g Vanadiumpentoxid und 112 g 105 %iger Phosphorsäure, welche einer Zusammensetzung von etwa 49 % Orthophosphorsäure, etwa 42 % Pyrophosphorsäure, etwa 8 % Triphosphorsäure und etwa 1 % höhere Polyphosphorsäure entspricht, unter Rühren in 1,5 L Isobutanol gegeben und die erhaltene Suspension für 16 Stunden unter Rückfluß erhitzt. Die Suspension wurde anschließend abgekühlt und filtriert. Der isolierte Niederschlag wurde 2 Stunden bei 150°C getrocknet und 1 Stunde bei 400°C unter Luft getempert.

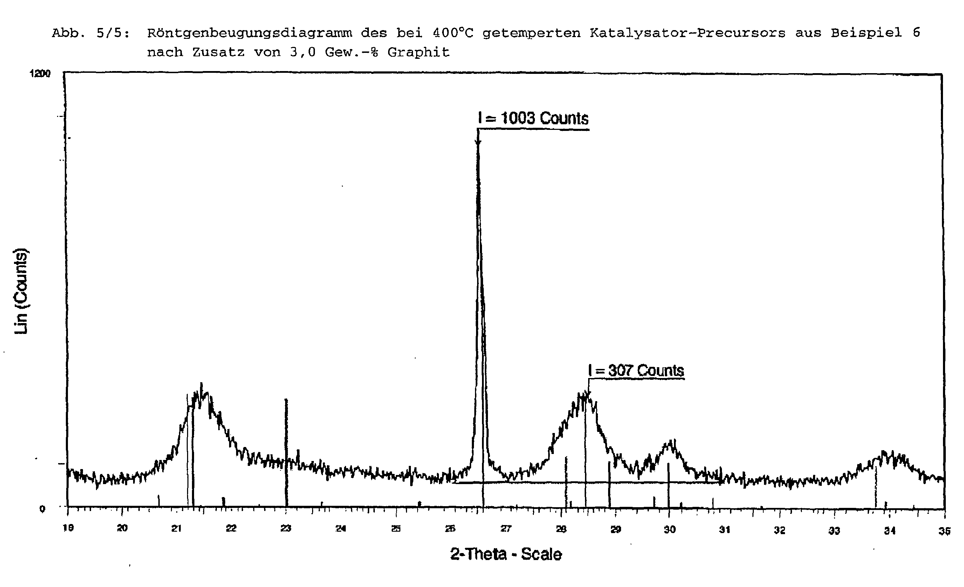

Der erhaltene Katalysator-Precursor wies eine BET-Oberfläche von 19 m$^2$/g auf. Die mittlere Oxidationsstufe des Vanadiums betrug 4,62. Ferner wurde eine Probe mit 3,0 Gew.-% Graphit vermischt und das Röntgenbeugungsdiagramm aufgenommen, welches in Abbildung 5 dargestellt ist. Das erhaltene XRD-Peakhöhenverhältnis I(28,5°)/I(26,6°) betrug 0,24. Aus dem relativ hohen XRD-Peakhöhenverhältnis ist ersichtlich, dass durch die Temperung bei 400°C bereits eine nennenswerte Umwandlung zum Vanadiumpyrophosphat stattgefunden hat. Des Weiteren traten unerwünschte $V^{+5}OPO_4$-Phasen auf.

Beispiel 7: Nachstellung von "Example 1, Part A" der EP-A 0 520 972 (Vergleichsbeispiel)

In Beispiel 7 wurde "Example 1, Part A" der EP-A 0 520 972 nachgestellt. Hierzu wurden 9000 mL Isobutanol, 378,3 g Oxalsäure und 848,4 g Vanadiumpentoxid vorgelegt, unter Rühren 997,6 g 105,7 %ige Phosphorsäure zugegeben und die erhaltene Suspension für 16 Stunden unter Rückfluß erhitzt. Nachdem innerhalb einer weiteren Stunde etwa 25 % des Isobutanols durch Verdampfung entfernt wurde, wurde die Suspension abgekühlt und etwa die Hälfte der verbliebenen Isobutanolmenge abdekantiert. Die verbliebene Mischung wurde in eine Schale überführt und 24 Stunden bei 110 bis 150°C unter Stickstoff getrocknet. Das getrocknete Produkt wurde anschließend 5 Stunden unter Luft bei 250 bis 260°C getempert.

Der Gesamt-Kohlenstoffgehalt des erhaltenen Katalysator-Precursors betrug 1,7 Gew.-%, der Gehalt an anorganischem Kohlenstoff 0,52 Gew.-% und der Gehalt an organischem Kohlenstoff 1,18 Gew.-%.

Wie das Vergleichsbeispiel 7 zeigt, wird nach der in EP-A 0 520 972 offenbarten Vorschrift ein Katalysator-Precursor mit Gehalt an organischem Kohlenstoff von über 1,1 Gew.-% erhalten.

Tabelle 4: Analysendaten der im Drehrohr getemperten Katalysator-Precusor

| Beispiel | 4.1 | 4.2 | 4.3 | 4.4 | 4.5 | 4.6 | 4.7 |
|---|---|---|---|---|---|---|---|
| Rest-Isobutanolgehalt [Gew.-%] | 0 | 0 | 0 | 0 | 0 | 0 | 0 |
| Gesamt-Kohlenstoffgehalt [Gew.-%] | 1,10 | 1,00 | 0,95 | 1,00 | 0,77 | 1,00 | 1,00 |
| Gehalt an anorganischem Kohlenstoff [Gew.-%] | 0,43 | 0,56 | 0,40 | 0,34 | 0,35 | 0,55 | 0,60 |
| Gehalt an organischem Kohlenstoff [Gew.-%] | 0,67 | 0,44 | 0,55 | 0,66 | 0,42 | 0,45 | 0,40 |
| I(28,5°)/I(26,6°) | n.b. | n.b. | n.b. | < 0,01 | n.b. | n.b. | n.b. |
| n.b.: nicht bestimmt | | | | | | | |

## Patentansprüche

1. Verfahren zur Herstellung eines Vanadium, Phosphor und Sauerstoff enthaltenden Katalysator-Precursors für die Herstellung von Maleinsäureanhydrid durch heterogenkatalytische Gasphasenoxidation eines Kohlenwasserstoffs mit mindestens vier Kohlenstoffatomen, **dadurch gekennzeichnet, dass** man

   (a) Vanadiumpentoxid in Gegenwart eines primären oder sekundären, nichtcyclischen oder cyclischen, unverzweigten oder verzweigten, gesättigten Alkohols mit 3 bis 6 Kohlenstoffatomen mit 102 bis 110 %iger Phosphorsäure in einem Temperaturbereich von 80 bis 160°C umsetzt;
   (b) den gebildeten Niederschlag isoliert;
   (c) im isolierten Niederschlag durch Temperung in einem Temperaturbereich von 250 bis 350°C einen Gehalt an organischem Kohlenstoff von ≤1,1 Gew.-% einstellt, wobei das getemperte Produkt nach Zusatz von 3,0 Gew.-% Graphit als inneren Standard unter Anwendung von CuKα-Strahlung ($\lambda$ = 1,54 10-1° m) ein Pulver-Röntgenbeugungsdiagramm ergibt, das im 2θ-Bereich ein Peakhöhenverhältnis des Peaks einer eventuell vorhandenen Pyrophosphatphase bei 28,5° zu dem vom Graphit stammenden Peak bei 26,6° von ≤0,1 aufweist; und
   (d) das aus Schritt (c) erhaltene, getemperte Produkt zu Partikeln mit einem gemittelten Durchmesser von mindestens 2 mm formt.

2. Verfahren nach Anspruch 1, **dadurch gekennzeichnet, dass** man in Schritt (c) die Temperung bei 270 bis 340°C durchführt.

3. Verfahren nach den Ansprüchen 1 bis 2, **dadurch gekennzeichnet, dass** man in Schritt (c) einen Gehalt an organischem Kohlenstoff von ≤0,8 Gew.-% einstellt.

4. Verfahren nach den Ansprüchen 1 bis 3, **dadurch gekennzeichnet, dass** das getemperte Produkt aus Schritt (c) nach Zusatz von 3,0 Gew.-% Graphit als inneren Standard unter Anwendung von CuKα-Strahlung ($\lambda$ = 1,54 ·10-10 m) ein Pulver-Röntgenbeugungsdiagramm ergibt, das im 2θ-Bereich ein Peakhöhenverhältnis des Peaks einer eventuell vorhandenen Pyrophosphatphase bei 28,5° zu dem vom Graphit stammenden Peak bei 26,6° von ≤0,05 aufweist.

5. Verfahren nach den Ansprüchen 1 bis 4, **dadurch gekennzeichnet, dass** man in Schritt (d) Partikel im Wesentlichen hohlzylinderförmigen Struktur formt.

6. Verfahren nach den Ansprüchen 1 bis 5, **dadurch gekennzeichnet, dass** man als primären oder sekundären, nichtcyclischen oder cyclischen, unverzweigten oder verzweigten, gesättigten Alkohol mit 3 bis 6 Kohlenstoffatomen Isobutanol einsetzt.

7. Katalysator-Precursor für die Herstellung von Maleinsäureanhydrid durch heterogenkatalytische Gasphasenoxidation eines Kohlenwasserstoffs mit mindestens vier Kohlenstoffatomen, erhältlich gemäß den Ansprüchen 1 bis 6.

8. Verfahren zur Herstellung eines Vanadium, Phosphor und Sauerstoff enthaltenden Katalysators für die Herstellung von Maleinsäureanhydrid durch heterogenkatalytische Gasphasenoxidation eines Kohlenwasserstoffs mit mindestens vier Kohlenstoffatomen, durch Behandlung eines Vanadium, Phosphor- und Sauerstoff enthaltenden Katalysator-Precursors in mindestens einer Atmosphäre, umfassend Sauerstoff ($O_2$), Wasserstoffoxid ($H_2O$) und/oder Inertgas in einem Temperaturbereich von 250 bis 600°C, **dadurch gekennzeichnet, dass** man einen Katalysator-Precursor gemäß Anspruch 7 einsetzt.

9. Katalysator für die Herstellung von Maleinsäureanhydrid durch heterogenkatalytische Gasphasenoxidation eines Kohlenwasserstoffs mit mindestens vier Kohlenstoffatomen, erhältlich gemäß Anspruch 8.

10. Verfahren zur Herstellung von Maleinsäureanhydrid durch heterogenkatalytische Gasphasenoxidation eines Kohlenwasserstoffs mit mindestens vier Kohlenstoffatomen mit Sauerstoff enthaltenden Gasen, **dadurch gekennzeichnet, dass** man einen Katalysator gemäß Anspruch 9 einsetzt.

**Claims**

1. A process for preparing a vanadium, phosphorus, and oxygen catalyst precursor for preparing maleic anhydride by heterogeneously catalyzed gas-phase oxidation of a hydrocarbon having at least four carbon atoms, which c;omprises

   (a) reacting vanadium pentoxide with from 102 to 110% strength phosphoric acid in the presence of a primary or secondary, noncyclic or cyclic, unbranched or branched, saturated alcohol having from 3 to 6 carbon atoms in a temperature range from 80 to 160°C;
   (b) isolating the precipitate formed;
   (c) setting an organic carbon content of $\leq 1.1\%$ by weight in the isolated precipitate by heat treatment in a temperature range from 250 to 350°C, the heat-treated product, following the addition of 3.0% by weight of graphite as internal standard and using CuK$\alpha$ radiation ($\lambda = 1.54 \cdot 10^{-10}$ m), giving a powder X-ray diffraction diagram which in the 2θ region features a ratio of the height of the peak of any pyrophosphate phase present at 28.5° to the height of the peak due to the graphite at 26.6° of $\leq 0.1$; and
   (d) shaping the heat-treated product obtained from step (c) into particles having an averaged diameter of at least 2 mm.

2. The process according to claim 1, wherein heat treatment in step (c) is conducted at from 270 to 340°C.

3. The process according to either of claims 1 and 2, wherein an organic carbon content of $\leq 0.8\%$ by weight is set in step (c).

4. The process according to any of claims 1 to 3, wherein the heat-treated product from step (c), following the addition of 3.0% by weight of graphite as internal standard and using CuK$\alpha$ radiation ($\lambda = 1.54 \cdot 10^{-10}$ m), gives a powder X-ray diffraction diagram which in the 2θ region features a ratio of the height of the peak of any pyrophosphate phase present at 28.5° to the height of the peak due to the graphite at 26.6° of $\leq 0.05$.

5. The process according to any of claims 1 to 4, wherein particles of substantially hollow cylindrical structure are shaped in step (d).

6. The process according to any of claims 1 to 5, wherein said primary or secondary, noncyclic or cyclic, unbranched or branched, saturated alcohol having from 3 to 6 carbon atoms is isobutanol.

**7.** A catalyst precursor for preparing maleic anhydride by heterogeneously catalyzed gas-phase oxidation of a hydrocarbon having at least four carbon atoms, obtainable according to any of claims 1 to 6.

**8.** A process for preparing a vanadium-phosphorus-oxygen catalyst for preparing maleic anhydride by heterogeneously catalyzed gas-phase oxidation of a hydrocarbon having at least four carbon atoms by treating a vanadium, phosphorus, and oxygen catalyst precursor in at least one atmosphere comprising oxygen ($O_2$), hydrogen oxide ($H_2O$), and/or inert gas in a temperature range from 250 to 600°C, which comprises using a catalyst precursor according to claim 7.

**9.** A catalyst for preparing maleic anhydride by heterogeneously catalyzed gas-phase oxidation of a hydrocarbon having at least four carbon atoms, obtainable according to claim 8.

**10.** A process for preparing maleic anhydride by heterogeneously catalyzed gas-phase oxidation of a hydrocarbon having at least four carbon atoms with oxygenous gases, which comprises using a catalyst according to claim 9.


## Revendications

**1.** Procédé de fabrication d'un précurseur de catalyseur contenant du vanadium, du phosphore et de l'oxygène pour la fabrication d'anhydride de l'acide maléique par oxydation en phase gazeuse sous catalyse hétérogène d'un hydrocarbure qui comporte au moins quatre atomes de carbone, **caractérisé en ce que**

(a) du pentoxyde de vanadium est mis en réaction en présence d'un alcool primaire ou secondaire, non cyclique ou cyclique, non ramifié ou ramifié, saturé, comportant 3 à 6 atomes de carbone, avec de l'acide phosphorique à 102 à 110 % dans une plage de températures allant de 80 à 160 °C ;
(b) le précipité formé est isolé ;
(c) une teneur en carbone organique inférieure ou égale à 1,1 % en poids est ajustée dans le précipité isolé par traitement thermique dans une plage de températures allant de 250 à 350 °C, le produit traité thermiquement donnant, après l'ajout de 3,0 % en poids de graphite en tant qu'étalon interne, en utilisant un rayonnement $CuK\alpha$ ($\lambda = 1,54 \cdot 10^{-10}$ m), un diagramme de diffraction de rayons X sur poudre qui présente dans le domaine $2\theta$ un rapport des hauteurs de pics entre le pic d'une phase pyrophosphate éventuellement présente à 28,5° et le pic originaire du graphite à 26,6° inférieur ou égal à 0, 1 ;
et
(d) le produit traité thermiquement, obtenu à l'étape (c) est mis sous la forme de particules d'un diamètre moyen d'au moins 2 mm.

**2.** Procédé selon la revendication 1, **caractérisé en ce que** le traitement thermique de l'étape (c) est réalisé à 270 à 340 °C.

**3.** Procédé selon les revendications 1 à 2, **caractérisé en ce qu'**une teneur en carbone organique inférieure ou égale à 0,8 % en poids est ajustée à l'étape (c).

**4.** Procédé selon les revendications 1 à 3, **caractérisé en ce que** le produit traité thermiquement de l'étape (c) donne, après l'ajout de 3,0 % en poids de graphite en tant qu'étalon interne, en utilisant un rayonnement $CuK\alpha$ ($\lambda = 1,54 \cdot 10^{-10}$ m), un diagramme de diffraction de rayons X sur poudre qui présente dans le domaine $2\theta$ un rapport des hauteurs de pics entre le pic d'une phase pyrophosphate éventuellement présente à 28,5° et le pic originaire du graphite à 26,6° inférieur ou égal à 0,05.

**5.** Procédé selon les revendications 1 à 4, **caractérisé en ce que** des particules essentiellement d'une structure cylindrique creuse sont formées à l'étape d).

**6.** Procédé selon les revendications 1 à 5, **caractérisé en ce que** l'isobutanol est utilisé en tant qu'alcool primaire ou secondaire, non cyclique ou cyclique, non ramifié ou ramifié, saturé, comportant 3 à 6 atomes de carbone.

**7.** Précurseur de catalyseur pour la fabrication d'anhydride de l'acide maléique par oxydation en phase gazeuse sous catalyse hétérogène d'un hydrocarbure qui comporte au moins quatre atomes de carbone, pouvant être obtenu selon les revendications 1 à 6.

**8.** Procédé de fabrication d'un catalyseur contenant du vanadium, du phosphore et de l'oxygène pour la fabrication d'anhydride de l'acide maléique par oxydation en phase gazeuse sous catalyse hétérogène d'un hydrocarbure qui comporte au moins quatre atomes de carbone, par traitement d'un précurseur de catalyseur contenant du vanadium, du phosphore et de l'oxygène dans au moins une atmosphère qui comprend de l'oxygène ($O_2$), de l'oxyde d'hydrogène ($H_2O$) et/ou un gaz inerte dans une plage de températures de 250 à 600 °C, **caractérisé en ce qu'**un précurseur de catalyseur selon la revendication 7 est utilisé.

**9.** Catalyseur pour la fabrication d'anhydride de l'acide maléique par oxydation en phase gazeuse sous catalyse hétérogène d'un hydrocarbure qui comporte au moins quatre atomes de carbone, pouvant être obtenu selon la revendication 8.

**10.** Procédé de fabrication d'anhydride de l'acide maléique par oxydation en phase gazeuse sous catalyse hétérogène d'un hydrocarbure qui comporte au moins quatre atomes de carbone avec des gaz contenant de l'oxygène, **caractérisé en ce qu'**un catalyseur selon la revendication 9 est utilisé.

Abb. 1/5: Untergrundkorrigiertes Röntgenbeugungsdiagramm einer repräsentativen Probe des im Muffelofen getemperten Katalysator-Precursors aus Beispiel 2.2 nach Zusatz von 3,0 Gew.-% Graphit

EP 1 487 577 B1

Abb. 2/5: Lichtmikroskopische Aufnahme des im Muffelofen bei 250°C getemperten und anschließend tablettierten und kalzinierten Katalysators aus Beispiel 2.1 (Vergleichsbeispiel)

V-Ox = 4,3

Abb. 3/5: Lichtmikroskopische Aufnahme des im Muffelofen bei 300°C getemperten und anschließend tablettierten und kalzinierten Katalysators aus Beispiel 2.2

EP 1 487 577 B1

Abb. 4/5: Untergrundkorrigiertes Röntgenbeugungsdiagramm einer repräsentativen Probe des im Drehrohr getemperten Katalysator-Precursors aus Beispiel 4.4 nach Zusatz von 3,0 Gew.-% Graphit

Abb. 5/5: Röntgenbeugungsdiagramm des bei 400°C getemperten Katalysator-Precursors aus Beispiel 6 nach Zusatz von 3,0 Gew.-% Graphit

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0520972 A
- WO 0072963 A
- EP 0056183 A
- WO 9712674 A
- WO 9526817 A
- US 5137860 A
- US 5296436 A
- US 5158923 A
- US 4795818 A
- WO 9301155 A
- US 5168090 A
- WO 0168245 A
- US 4933312 A
- WO 9529006 A

**In der Beschreibung aufgeführte Nicht-Patentliteratur**

- MALEIC AND FUMARIC ACIDS, Maleic Anhydride - Production. Ullmann's Encyclopedia of Industrial Chemistry. 2000
- **B. Kubias et al.** *Chemie Ingenieur Technik,* 2000, vol. 72 (3), 249-251